# EUROPÄISCHE PATENTSCHRIFT

(11) **EP 2 326 713 B1**
(45) Veröffentlichungstag und Bekanntmachung des Hinweises auf die Patenterteilung: **25.11.2015**
(21) Anmeldenummer: 09775638.1
(22) Anmeldetag: 18.09.2009
(51) Int. Cl.: C12N 9/10, C12N 9/18, C12N 15/52, C12P 7/06, A23K 1/165

(54) **VERFAHREN ZUR HERSTELLUNG EINES ZUATZSTOFFES FÜR DEN ENZYMATISCHEN ABBAU VON MYKOTOXINEN SOWIE ZUSATZSTOFF UND VERWENDUNG DESSELBEN**
METHOD FOR THE PRODUCTION OF AN ADDITIVE FOR THE ENZYMATIC DECOMPOSITION OF MYCOTOXINS, ADDITIVE, AND USE THEREOF
PROCÉDÉ DE PRÉPARATION D'UN ADDITIF DESTINÉ À LA DÉGRADATION ENZYMATIQUE DE MYCOTOXINES, ADDITIF ET UTILISATION DE CELUI-CI

(30) Priorität: 18.09.2008 AT 50108 U
(43) Veröffentlichungstag der Anmeldung: 01.06.2011
(60) Teilanmeldung: 15000331.7
(73) Patentinhaber: Erber Aktiengesellschaft, 3130 Herzogenburg (AT)
(72) Erfinder: MOLL, Wulf-Dieter, A-2000 Stockerau (AT); HARTINGER, Doris, A-1220 Wien (AT); GRIESSLER, Karin, A-3140 Pottenbrunn (AT); BINDER, Eva Maria, A-3430 Tulln (AT); SCHATZMAYR, Gerd, A-3430 Tulln (AT)
(74) Vertreter: Cunow, Gerda
(86) Internationale Anmeldenummer: PCT/AT2009/000364
(87) Internationale Veröffentlichungsnummer: WO 2010/031101

(56) Entgegenhaltungen:
- WO-A1-00/04160
- WO-A2-00/04158
- WO-A2-2004/085624
- WO-A2-2006/053357
- Hartinger D. et al.: "Heterologous expression of genes from the fumonisin degradation gene cluster of Sphingomonas spp. MTA144 and activity of the catabolic enzymes" New Biotechnology 13. August 2009 (2009-08-13), XP002566822 Gefunden im Internet: URL:http://dx.doi.org/10.1016/j.nbt.2009.0 6.444> [gefunden am 2010-02-04]
- Heinl S. et al.: "Identification of a fumonisin degrading gene cluster in Shipngomonas spp. MTA144" New Biotechnology 13. August 2009 (2009-08-13), XP002566823 Gefunden im Internet: URL:http://dx.doi.org/10.1016/j.nbt.2009.0 6.290> [gefunden am 2010-02-04]
- HEINL S. ET AL.: "Degradation of fumonisin B1 by the consecutive action of two bacterial enzymes" JOURNAL OF BIOTECHNOLOGY, Bd. 145, Nr. 2, 15. Januar 2010 (2010-01-15), Seiten 120-129, XP002566276

## Beschreibung

Die vorliegende Erfindung bezieht sich auf einen Zusatzstoff zum enzymatischen Abbau von Fumonisinen, in einem pflanzlichen Rohstoff und Mischungen, die pflanzliche Rohstoffe enthalten, sowie eine Verwendung desselben.

Mykotoxine treten auf landwirtschaftlichen, pflanzlichen Produkten sehr häufig auf und verursachen je nach Art der Mykotoxine schwere wirtschaftliche Schäden, insbesondere in den aus den landwirtschaftlichen Produkten hergestellten Nahrungsmitteln und auch bei mit derartigen Nahrungsmitteln ernährten Tieren und Menschen, wobei derartige Schäden äußerst vielfältig sind. Es wurden bereits zahlreiche Methoden entwickelt, mit welchen versucht wird, derartige Mykotoxine zu entgiften bzw. abzubauen oder unschädlich zu machen, um durch die Mykotoxine verursachte Schäden in den Bereichen von tierischer und menschlicher Ernährung, der Tierzucht, Verarbeitung von Futter- und Lebensmitteln und dgl. hintanzuhalten.

Unter den bekannten Mykotoxinen existiert eine Vielzahl von untereinander strukturell verwandten Mykotoxinen, wie beispielsweise die Fumonisine, von welchen Fumonisin B1 das am häufigsten vorkommende Toxin der Gruppe ist. Jedoch sind zahlreiche Derivate und verwandte Moleküle bekannt, welche ebenfalls giftige Wirkungen bei Menschen und Tieren aufweisen. So ist es bekannt, dass die Fumonisine den Sphingolipidstoffwechsel durch eine Wechselwirkung mit dem Enzym Ceramidsynthase behindern. Sphingolipide sind nicht nur Bestandteil von Zellmembranen, sondern spielen auch eine wichtige Rolle als Signal- und Botenmoleküle in vielen elementaren, zellulären Prozessen, wie dem Zellwachstum, der Zellwanderung und Zellanbindung, bei entzündlichen Prozessen und intrazellulären Transportvorgängen. Aufgrund dieser Behinderung des Sphingolipidstoffwechsels werden Fumonisine für die giftige Wirkung auf unterschiedlichste Tierarten und auch für den Menschen verantwortlich gemacht. So konnte nachgewiesen werden, dass Fumonisine bei Nagetieren krebserregend wirken, und sie wurden durch epidemiologische Daten mit Speiseröhrenkrebs und dem Neuralrohrdefekt bei Menschen in Zusammenhang gebracht. Bei verschiedenen Tierarten, wie beispielsweise Schweinen, werden sie für die typische Toxikose durch Lungenödeme verantwortlich gemacht. Fumonisine sind in diesem Zusammenhang eine nahezu allgegenwärtige Kontamination auf verschiedensten Getreidearten und insbesondere auf Mais sowie auf Nüssen und Gemüse sind, ist dieser stark negative Effekt in Bezug auf die Gesundheit von Menschen und Tieren nicht vernachlässigbar.

Der mikrobielle Abbau von Fumonisinen wurde bereits in der EP-A 1 860 954 beschrieben, gemäß welcher Mikroorganismen zur Entgiftung von Fumonisinen und Fumonisinderivaten eingesetzt werden, bei welchen die detoxifizierenden Bakterien oder Hefen, gewählt aus genau definierten Stämmen zur Entgiftung von Fumonisinen, Futtermitteln zugesetzt werden.

Auch wurden bereits katabolische Stoffwechselwege für den biologischen Abbau von Fumonisinen und die hiefür verantwortlichen Gene und Enzyme beschrieben. So beschreibt beispielsweise die EP 0 988 383 Fumonisin entgiftende Zusammensetzungen und Verfahren, wobei die eingesetzten, Fumonisin abbauenden Enzyme in erster Linie in transgenen Pflanzen produziert werden, bei welchen die Entgiftung von Fumonisinen mit Hilfe einer Aminooxidase, welche für ihre enzymatische Aktivität molekularen Sauerstoff benötigt, erfolgt.

Des Weiteren beschreibt die WO 2004/085624 Transaminasen, Deaminasen und Aminomutasen und Zusammensetzungen und Verfahren zur enzymatischen Detoxifizierung, wobei insbesondere aminierte Toxine, beispielsweise Fumonisine, entgiftet werden. In diesem Zusammenhang werden Polypeptide, welche eine Deaminaseaktivität besitzen, zur Entgiftung eingesetzt.

Aus der WO 00/04158 ist die Verwendung von Fumonisin abbauenden Aminooxidasen bei der Herstellung von Nahrungs- oder Futtermitteln bzw. bei der Verarbeitung von pflanzlichen Rohstoffen bekannt geworden.

Bisher bekannten Verfahren ist jedoch gemeinsam, dass sie für eine Detoxifizierung der Mykotoxine molekularen Sauerstoff für die beschriebenen, katabolischen Stoffwechselwege benötigen, wobei die insbesondere erforderlichen Aminooxidasen unter sauerstoffunabhängigen Bedingungen nicht arbeiten können. Ein Einsatz von derartigen Genen und Enzymen zur Detoxifizierung von Futtermitteln, beispielsweise im Verdauungstrakt von Tieren, ist aufgrund des im wesentlichen sauerstofffreien Milieus in dem Verdauungstrakt von Tieren nicht möglich bzw. zeigen die bekannten Gene und Enzyme keinerlei Wirkung.

Die Erfindung zielt nun darauf ab, einen Zusatzstoff für den enzymatischen Abbau von Mykotoxinen zur Verfügung zu stellen, mit welchem es sicher und zuverlässig gelingt, Fumonisine sauerstoffunabhängig zu toxikologisch unbedenklichen Substanzen abzubauen bzw. zu entgiften.

Als pflanzliche Rohrstoffe werden hiebei Getreide bzw. Getreideprodukte, Gräser, Obst oder Gemüse sowie Zwischenprodukte des diese Stoffe zur Herstellung von Nahrungs- und Futtermittel enthalten, wie beispielsweise Silage, Obstmaische oder dgl. verstanden.

Als Zusatzstoffe werden vor allem Futtermittelzusätze, Nahrungsmittelzusätze sowie Zusätze zur Bioethanolherstellung verstanden.

Die für den Abbau der Fumonisine eingesetzten Nukleinsäuresequenzen bzw. die mittels dieser Nukleinsäuresequenzen in prokaryotischen und eukaryotischen Wirtszellen exprimierten, in sauerstoffunabhängigem Milieu katalytisch wirkenden Enzyme sind nachfolgend aufgelistet.

### Nukleinsäuren

### Sequenzen:

>Seq ID 1 (fum (Fumonisinkatabolismus) Gencluster, 15.420 bp) 
TGTCGGCGATCRGTAAACTTCTACCGTGGTCCTCGTTCGCCCACAKCATACATCACAGACRTCGGGATTTCCAACTGAAC
GGGTCCCGGCCTGCCGGCCCACATTTCCCGGAACGCCATATGGGTGATTTCGACAATCCGGTTCCAGGCGAAGATGGGTG
CGCCCCATTTAACCGCGGGTCGAAAGAGGTCGATCTGGTCTTGTCCCTGAAAGGTTTTTGGCGTGCAGGGATAAACGACA
CCAAGTTGATGCTGGGACGTTATTGCGACGAAGGGAACCCCTTCGTGGCGTGCCGTCACGACTCCAGGCAGAAGGTTTGC
CGTACCGGGACCCGGATTCGTGACAATCGCGGCGACCTGTCCGGTGGTCTTGTAAATGCCCTCGGCCATATAGGCTGCGG
CGGCCTCGTGCCGCACCGGGACGAACAATATCCCATTGTCTTCGAGCGCAGCCAGGAGCGGATCCACCTCCGGCGACATG
AGGCCGAAGACATACCGGACGCCTTCGACGGCCAAACATCGTGCCAATAATTCTCCGCCCGTGAGGCGCATGACGATCTC
CAGTACGAAAGGTGAGTGCCCAGGTTCCGGCACATTCGCTGTGGTTAGTTGATGCGCTGATCGGCCAACCGACTGAGTGG
AGTTGGATGGCCGCACCTTACCCTGTCGCGCATAACTCTCAGATCCGGAAACGGACCCCGACATTAAAATAGCGGCCGAC
CGGATCATAGGCAGAGCTGGTCGGCTGGAAAAACTGCTGGGGTCGTTCGTCGCTATTGGCGGATCTCGGTCGAACAAAT
TATTGACCGATAGAAACAGCTGCTGCTTCTGGCCAAAAGCCGCGATGTCGAAGGTCAATGTCGCGTCGGTGTACCAAACC
GCCGGAGCGTGGTTCAAATTCGTATCGACGCCCTCCACATTGTCGGCATTGAACACCGATGCTGCGATGAAGCGCTGCTG
CACGAGAAGCGCCCAATCGTCGGTCGAATATCGCGCCTGGAAGTTGGCCGACCATTTTGGCGTGTCCGGTTGTCCGAGCG
AACGGATGGCGCCGAGCCGGTCGCGATGCGATAGGCAGAGGTATGGTGCGTTGCCAGCGCACGAAGACTGAACGTGCCG
CCGCCGACGGGGCGTGAGTAATAGGCCTCGAAGTCAATTCCCGCCGCTTTCTGGACAGCCAGGTTGAGATTGGGACCCGT
CACTGTGATGGTGCCGTCCGGATTCTCCGTTATGAGGTCGCAGAAGAAGGTGTTTCCTGCATCGCACGCGTCGATTTCCT
GCTGGGGAAGGAGGAAATCGATCGCGCCCTTCACCTTCACCACATAGCGATCGACCGAAAACTGAAACCCCGGCACGAAG
GCGGGGCGTAGCACCGCGCCGAATGTAAGGACGTCCGCCTTTTCAGGGCGCAAATCCGCGTTGCCGGCGGTAAAGAACCG
CGTCTGCACAGCCTGTCCGCCATAAATTGAATTGAGCGTCGCCTGACGGCCGGGGTCGAATAGCTCGACAAGGCTTGGCC
CGCGGATATCTCGCGAACGGGTCGCGCGGAACCTGAGGCCGTCGATCGGCTCATATTCTCCGCCCAGCTTCCAGGTTGTT
ACTCCACCGGACTGGCTGTAATCGGCATATCGGACGGCGCCGTTTAAGTTCAGCGAACGTCCCAGCGCGCTGTCCTTCAG
AATCGGGACGCCGATTTCGACAAAACCTTCCTTGATGTCATAGCTTCCCGAGAAGGGAAGTGGGTTGTAGAGATTGAAGC
CTCCAGGCCGACCTGCCTGCGCCGCCGGAGCCCCCCTGATTCCCGTGATCGAGGTCGTCGCCTGCGATATCGCGTCGGTT
TCCTGCCGGGCCTTCTCCTTGCGATATTCGATACCAGCGGCGACCGAGACCGGGCCCGCGCCGAACGACAGGCTATCGCC
GAGGTCGCCGGAAATCGTGAGTCCCGCCACATATTGCTCAAGCCTCAGCTGAGCGACGCCATCAGCGGTGACATAGTCGA
TGGCCGACGCGCTCGGCGAGCCTGTGCCGAAGAGATTGAGCGGCACGCAATCTTGGTCGAGGCCGGCCAGTGTTGAACGG
CAGACGATATTGCCCGCGGGATCGCGGACCGCATCGACGGCGGCGTAGAGATTGCGGTTGATGGTGAGATTGTTTTCACG
AAGCTCGAGGTCCGTAAGGCCAAAGGAGGCCGAGCCATCGAGTTTCCAGCCATTGCCAATGTCTGCCCGGAAGCCGGCAG
CGCCGCGGTAGACCTTTGCGAAATTCTCGATTTCGACCAAGGGAAAGTCGCTTGAGAAGCGACCGACAACGATCGAAGCC
TGGGCATTTCTGTCCATGAGCGTCGCGAGTGGAGCCGGAAGGAAGGCGTTATCACGGAAGATCCGGAAATTATTCGAGCC
ACCGACATGCGATATTACGAATGCACCCAGGTTGGTGTGGGAATAAGCATAGGTGCCCTCCGCATACACCTGCACAGTGT
CGGACACATCATATGCGGCGCGTAGGAACGCGTTGTAGCGAAGCTGATCCGGGGCGAAGCCGATATTCACGCGCGGTCCA
TCGCCGCCGCTCTGGAACGACGAGCTCGTAAAATTCCCGTAGTCGAAGGTCCCTAGGACTCCTCCGGGCAAAAACGCGAT
GCCTTTCAGAGGGCCGGACGTGACAAGTCCGCCGTAGGATCCGCGAGAACTGCGAATATCGGGCACGACCGTGACGCCTG
TCGTAGCGCCGGGCACGGGATATTGGCCGGCGGCGATGTCGAACCAGCGGCGACCCGTTGCTTCATCGGCCCGGATTCCG
TCCTGTCGAAAATATTCGAAGCTGCCGAGCAAGTGCAACCGGTCGTCGGCAAACGAAGTGCCGAAGGCGATCGAACCGCC
GTAGGACGGGAGGTCGCCGCGGGTTGAAACACCCGACTGGAGCTCGGCCCTGATGCCTTCCAGATCTTCGTCGAGCACGA
 
AGTTGATGACGCCCGAAACGGCATCGGAACCGTAGGCGGCCGAGGCGCCGCCCGTCACGACATCGACGCGCTTGACCAAC
GCCTGCGGCAGCACGTTGATATCGACCGAGCCTGTGAAATTGGTCGCGACGAAACGGTTGCCGTTCAGCAGGACGAGGTT
CCGGTTTGACCCGAGGCCGCGCATGTTGAGCAGGTTCTGACCGCTGTTCCCCGTTCCGGGTGTCGTGCCAGGGTTGGAGG
TCTTCAAGCTGTCGTTGAACACGGGCAGCTGGTTGAGTGCGTCGGCAAGGTTGGTCGGAGATGCCTCCTTCAACTGCTCG
CTGGATACGGCTGTAACCGGCGTCGGCGAATTGAAGCCGTTCTGGAGGCGGCTGCCGGTCACGACGATTTCGCTCGTTCC
CCGGTCCGTGTCCGCTTCGTCCGGCTGACCTATCGATGCGGATCGCTATCCTGAGCACTGGCAGAGACAGGAAATGCGA
GGGTGCCGAGCGCTACTGCGCCGAGCAAACTATTTGCCTTGCCGGGCTTTTCGATTCTGAACTTCCGATACATCTGCAGT
CCCTCCCGAATTGATAGGGACTCCGTTTGAGTCCCCTTGTTTCTTGACGCCGCCGTCGCTCACCACGGTCCGGTCGGAGG
CTAAGCGTCGGGCCTAAGGACCCGCAATTTGAACATCAAATGCAATGATCGGAGGCTTCATTGCACTTCGCGCATAGACC
GGCGCGGTAGCTGAAAGTGCCAATAATCAGGGATTTTGCTGAACAGTTGCGGCATGACGTCCGGCATCGGCCACGCGGTT
GGCGGCATCGACGTGGCTTTCGCGTCGCCGCCCCTCAAGCACCGGCGAGTTGCATTAAAATGGGATGAGGCTGGAGAGAC
GCAAAATCTCTGAGGACCGCGCTGAACGCGCGATCCGTCGCCTCGAGGGTCTCCGTTACATCGTCAACTGTATGGGCCGC
AGAGAGAAACATATTGTGATAGGGATGAACATAGACGCCGCCCTTCAGGCACGCCGCGGCCCACGCATAGCCGATCCGAA
AATCGGGATCGTCCGCAAAGAATATTTGCGGCATCTGCGCCGGGCCCGTCTGCTTCAACTCAAGACCATGGCGCTGAGAC
TGTGCCTCCAGGCCTGCCCGCAGGGCGGCGCCGCTGGCGATCAGCGTTTCGAGATAAGGCGTCTCTCGAATGATCCTGAG
GGTTTCGATCGCGGCCGCCATCGGTACCGCAGAGAACCAGAAGGAGCCGGTCACAAATATATCCCGCGCCGCATCGCGCG
CCTTGTTCGAGCCCAGCAGGGCGGAGATCGGATAGCCATTCGCAAAGCATTTTCCCCAGCAACTGAGATCGGGTTCGATA
CCCAAATGCGTCCAGCTGCAATCGCGCGCCACCCGGAAACCTGCGCGCACATCGTCAACGACCAGAAGCGCACCGGTCTC
GTCACAACATTTTCGAGCGGTGCGCGCGAACTCAAGCTGGGCGAGGGCCTGGTCCTCAAATACTTCGTGTCGGAAAGGTG
TGGCAAAGACAGCCGCAATATCGCCATCGTGCGCCTTGAACGCGTCCGATAAGCTTTGGGCGTCGTTATAGGTATAATAT
GCGACATGCACGCGATCGGAAGCGAGAATCCCGGCAGTATGCGGAGTGTTCCACGGGAAGCGCCATGATAGGCGCCTTT
GGCGCATAATATGGTTTTGCGCCCCGTATGGGCACGCGCGAGAACCATCGCCGTTGAGGTGGCATCGCTGCCATTTTTGC
AGAACATCGCCCAATCCGCATGACGGACCATGCCCACAAAGGCTTCGGCGAGGTTGACCATGATCTCCGAAGGACCGGTC
ATGGTGTCGCCGAGAAGTCGCTGCGCATCAGCCGCGGCTTCGATTTCGGATTGCCGGTAACCGAGCAAATTTGGCCCATA
CGCGCACATATAGTCGATATAGGGCTGCTCGTCGGCGTCCCAAATTCGTGCCCCCAGCGCGCGCCTGAAGAACTGGGGA
ATTCTGGCGGCAGCAACCGTGTCGACTCGTGGCCGTACATCCCGCCCGGAATGACCCGTTCGGCGCGTTCTCTGAGATCT
TTCTGCCTTGTTCCGTTCGCCATAATGCACCTCTCGCGATAAATAATGGGTAAAAATCCACGAAATTCAACGATTCGTGA
TCTGAAAGAGATATATCTTGTAATATACTGTATAATTATACACAATGCGCAATCGGACGACGGGATAGCGGGGCAGGGAG
GACGGGGAAATCTATGCGGAACGTCAGCGACAAGGCGCCGCCCCACGAGACGCTCACCGTAGTCGTCGCGGCAATGATCG
TTGGCACGGCCGCCTTGATGGTGCTTGGAATACAGCCCATCCTTCTCGGCGCCCTTGTAGAGGAGGGCGTATTCCCGCC
GAGGGGTTGGATCGGCGGCAACGGTGGAAATACTGGCGATCGCGGCGGGAACATGCATCGGACCCGTTCTTATGAAGAC
GGGATATCTGCGGGCGAAATGCGCGGCACTCTGCTTAATGCTCGCCCGCAATCAACTTCGGATTGACGTTGCCGGGTTTCG
ATTTGCCCATCGTGGCTTGCCGAGCGGCAGCGGGAGCCCTGGAAGGTCTTTCGCTCAGCGCGGCGATCCTGATCATGACT
CATAATCGGCGGCCGGACCGGCTGAGCGGAATATTTCTGGGCGCGCAGACGATACCGCAGGTAATATCTGCTTATTTGCT
CCCGACGGAGATTATTCCGCGCTGGGGGAGCGCAGGCGGCTTCACGATCCTGGGCATTCTCGCGGCGATCGCCGCGATCG
CGGCTCTGTGCCTCGTCGATCGCGTTGAGCTCGATCCGACGACCGTTAACGACGACTTGCAGTGGTCACCCGCGGCGATC
GTCATTTCGATGGCGGCATTCGTTCAATTCTCGGGGGTCGGTGCCGCATGGAGCTATCTGGAGCGACTGGCTGCGCAGCA
CGGATTTTCGGGAGAAACGATCGGTATCGCCATTTCCGGGAGTTTGCTTTGCCAGGTAGGCGGGGCTTGGCTGGCCGCTT
GGATCGGTGGGCGGGTCGGATATCGCTTCGCCTTAATCGCTGGGAGCCTGCTTCAGGCGGGCAACGTGATCGCATTGGCG
GTGGCCGATCAGCCAAGCTGGTTTATTTCCGCTTCCTGTGCTTTCGGCCTGTTCTGGTTGGCGATGCAGCCCTTCCAAAT
CCGCTTCGCGATCGCGATAGATAACAGCCGGCAGCTTGCTGTACTGCTGACGCCGATCGCCCTCGTCGGGTTGAGCGCGG
GGCCCTTGTTGCTCTCTCGCTTTGCCGGGGCGACCGACTTGCGCTGGATCTTTGTGGGGAGTTCGACCTTGTTGCTGGCC
AGCGCGCTTCTGTATCTTTGCGCTTCTCTGTTCAACCGCGCGGAAAGGTGATCGCTGAAACGGTGGACGTATGAAAAAG
ACGGATCGGGGTTCGCGATGACATCGCAGGTCAAGCTTCGTAGCGCGGCAAAGCGGCCGCGCAGTCCTAAAAGCGAGCGA
GGTCTTGCTCGTTACGAGTCCTTGCTTGATGCGACCGACAGGCTGTTGGTCGATCTAGACCCCGATCAGGTCGGTCTCTA
TCAGATTGCAGAGGAAGCGGGTGCCTCACCGTCGTCCGTCTATCATTTCTTTCCGACCAAGGAAGTGGCTCATCTCGCTC
TGATGCGCCGCTATCTGGAGGGGCTCCGGAATCTCGACGCGATGGAAGTCGACATCGGCCAGCTCGAAAGCTGGCAGGAC
CTGATGAAGTTGGATCAGATCAGGGCGCGAGACTATTATAATAGCCACCCGCCCGCCCTCAAGCTTCTGTTCGGCGGATA
TGGCGGGGTCGAGGCCAGAAAGCTTGACGAGCGATACTCCGAGGAAATCGTGAGCTCCATGTATGGCAGATACAACGGCA
TTTTCCATATGCCGCAAATGGAGAATGAGGCTCTCATGTTCACGATCTGCTTCGCAATTCTCGACGCGGTATGGGCCGTC
TCCTTTCGCCGGTTCGGTGAAATTACGTCGGATTTTCTTCGGGAGGGGCAAGCGGCTTGCATTGCCTATTGCCGACACTA
TCTGCCCGAGCGAACGCCATCAGCGTGAATCCGTTCAACGATATGCAGGAATGTCCGTTGCGTTGAGTTCGGTTCTGAGT
TCGGTCGGTTAGGAGGCCCCGCGATAAACCAACGCTCTTCTGTCGAAGGGATGTCGCCTGGTTCGACCAGGCCCTGCGAA
GTCAGCCGCAATCAACGAGGCAGATGTCAACGTGGCCAGCAAGTTCAACTGTGAGTTACTCGATCTGCGATCATTTGTTG
CGGTGTATGAAACGCGAAGTTTTAGCCACGCCGCGCGGCTTCTGAATCAATCGCAGCCCGCGCTCAGCCGGAGAATCCAG
CGCCTCGAGAGTCTCGTGGGCGGTCCGTTGTTCGAGCGGACCAGTCGGTCGCTTGCCGAAACGGCGCTCGGCAAAGAGTT
GCTCCCGGTCGCCCACCGAGCGTTGGAACTTGTCGATACGTCGCTGTTTGCGTCGCCCAATGTCCGGGAGTTCCGCTGGA
CAGACATCACGATTGCCTGTGTACAGACCGCCGCCTTCCATGTTCTCCCGCGAGCTGCGCGCTTGTACATGGATCAAAAT
CCGAGGGTCCGACTCCGCATCCTTGACGTGCCGGCGGTCGAGGCTGCGGACCTGGTTGCGAGCGGCGAGGCGGAGTTCGG
CATCAGCATTGAGAGCCTGTTGCCATCAAGCCTGCGGTTCGATGCGCTCCACGAGGACCCGTTCGGCCTGGCATGCCACC
GAAGCCATCCGCTGGCGTCGCTCGAGATCCTTGAATGGACGCAATTGAAAGGTGAAAGCCTGATCGCCGTTCACCGTGCG
AGCCGGAACCGCACGTTGCTCGATGCCGAACTCGCGCGCAACAATATCGCGCTGGAATGGCGGTATGAGGTCGCGCATCT
GACGACGGCGCTGGGATTGATCGATGCGCAATTGGGTGTCGCTGTTATGCCCCGCATGGTTATGCCCCGCTCGGGTCGGT
CGGAGGTCGTCTGGCGCCCCGTCGTCGCGCCGGTCGTCCAACGCACGATCGGCATCGTTCAGCGCCGCACCGGCTCGATG
CACCCTGCCGCACAGCAATTGCTTGCGCGGCTCCGCGCGGCCTGGTCGTCCGCCAATCTGGGCGACATCGCGTCTCGCGA
AGATGGGGCATCGTGACACGCGTTCTATGCGCCTGCAGCATCGATGCTCACGATCATTGCATTTGCTGAGAGACGAACGC
GAAGATACCGCTGGGTCACAGGATATCAGTCCATCGAGGCGGGAGAGAAATGTGTGAAAGAGCACCAATGCCGTGGCGGC
CGGGCGTCCCCCGCTGCGCCCGCCACGTGGCTTGCGCGGATCAGCGTTTCCCGGGGGGCCTCCGCCATCGCCTGGACCTT
CATGCTTGGCGCAACTGCCATTCCCGTGGCTGCGCAAACTGACGATCCGAAGCTCGTTCGTCATACCCAGTCGGGCGCCG
TCGAGGGCGTCGAGGGCGACGTCGAGACTTTTTTGGGAATACCCTTCGCGGCTCCGCCGGTCGGCGACCTGCGATGGCGG
CCGCCGGCTCCGCCGAGGCGTGGGCGGGCACCAGGGACGGCCGCCGCTTTGCGCCCGATTGCATCGGGAACGAGCGGCT
TAGAGAGGGGAGCCGGGCTGCCGGGACGAGCGAAGACTGCCTCTATCTGAATATCTGGTCTCCCAAACAGGTCGGTAAGG
GGGGGCTCCCCGTCATGATCTGGGTTTACGGCGGTGGGTTTAGCGGCGGTTCTGGCGCGGTGCCATATTATGACGGCTCT
GCGCTCGCGCAGAAGGGCGTGGTGGTCGTCACGTTCAACTATCGCGCCGGGATTCTGGGCTTTCTTGCCCATCCGGCGCT
TTCAAAGGAAAGTCCGAATGGCGTGTCGGGCAACTATGGTCTTCTCGACATGCTCGCGGCGTTCAAATGGGTTCAGAACA
 
ACATAAGGGAGTTCGGCGGAGACCCGAACCGTGTCACGGTCTTTGGCGAGTCCGCCGGCGCGAGCGCGCTCGGACTGCTC
CTGACCTCGCCGCTCAGTGAGAGCGCCTTCAATCAGGCGATACTGCAAAGTCCGGGTCTGGCCAGGCCGCTCGCCACGCT
TTCTGAAAGCGAAGCGAATGGGCTGGAGCTGGGAGCCGATATTTCTGCTCTACGGCGTGCCGATGCGGGCGAATTGACGA
AGATCGCGCAATCGCGAATACCCATGTCGCGCCAGTTCACCAAGCCGCGGCCGATGGGTCCGATTCTGGACGGCTATGTT
TTGCGCACCCTTGACGTCGATGCCTTCGCCAAGGGGGCCTTCCGCAAGATACCCGTTCTGGTCGGCGGAAACGCCGACGA
AGGGCGCGCTTTTACGGATCGCCTGCCGGTCAAAACGGTCCTTGAATATCGAGCCTATCTCACAGAACAATTTGGTGACG
AGGCGGACGCATGGGAGCGTTGTTATCCCGCGAACTCCGACGCCGACGTCCCCGCCGCCGTTGCCCGTCTTTTTGGGGAT
AGTCAGTTCAACAACGGGATCGAGCTGCTCTCGGCAGCCTTCGCGAAATGGCGAACGCCGCTTTGGAGATATCGCTTTAC
GGGCATTCCAGGAGCCGGCCGTCGCCCCGCCACGCATGGAGACGAAATTCCCTATGTCTTCGCAAATCTGGGGCCGTCGT
CCGTATCTATGTTTGGGTCGCTCGAAGGCGGCGCCGGGGCGTCGGACATCAAACTTGCGACCGAAATGTCCGCGGCCTGG
GTGAGCTTCGCGGTGCACGGGGTCCCCGATCAGGGCACGAAATCGCACTGGCCGCGCTTCGAGCGGCGAGGGGAGATCAT
GACTTTTGGTTCGCAGGTTGGCTCTGGGGAAGGTCTTGGAGTTTCGCCGAGCAAAGCCTGCCAACCCTCAAAATAGCGCC
CGGCCTGTGCGTGCTTCAGCACGCCGTCCCGCTTTGCGGGCGACGGGCTGTGCCCTCTGCCTAGAAGGAAGTAAGTTGCG
CTACGACGTCGCGATAATTGGAGGTGGCAACGCTGCATTGACGGCAGCCGTGACGGCGCGTGAAGCGGGGGCCTCGGTTC
TTGTGATCGAGCATGCGCCGCGCGCCATGCGCGGCGGCAACAGTCGTCACACACGCAATATGCGTACGATGCACGAACGT
CCCCTGTCGCCGTTGACCGGTGAATATTCGGCGGACGAATATTGGAATGATCTTGTCCGCGTCACGGGGGGCGCACCGA
CGAAGAACTCGCGCGGCTCGTTATCCGCAACACCACCGACGCTATTCCCTTCATGACGCGCTGCGGTGTGCGTTTCCAGC
CCTCGCTGTCGGGCACGCTGAGTTTATCGCGAACCAACGCATTCTTCCTTGGCGGCGGAAGGCGCTTGTAAACGCATAT
TACGCCACGGCCGAACGGCTAGGCGTCGATATTCTCTATGATTCTGAGGTGACCGAGATCAACCTTCAGCAAGGCGTCGT
GCAGCGTCTGCAATTGCGCAGCCGGGGATTCCCTGTCGAAGTGGAAGCCAAGGCTGCCATCGCCTCGTCCGGAGGATTCC
AGGCAAATCTTGACTGGCTCTCAAGCGCATGGGGGCCTGCTGCGGCGAACTTCATCGTACGGGGCACGCCATATGCGACT
GGCACGGTGCTCAAGAACCTGTTGGAGCAAGGCGTCGCCTCGGTGGGAGATCCAACCCAATGCCATGCTGTCGCGATCGA
TGGGCGAGCGCCCAAATACGACGGCGGCATCGTCACACGACTGGACTGCGTTCCCTTCTCGATCGTCGTCAACAAGGACG
CCTTGCGCTTCTACGATGAAGGCGAAGATGTGTGGCCGAAGCGTTACGCCATATGGGGTCGCTTGGTGGCACAGCAGCCT
GATCAGATCGCTTTCAGCATAATCGATCGGCAGGCCGAAGACCTCTTCATGCCGTCAGTGTTCCCCCCCGTGCAAGCGGA
CACGATCGCGGGTCTGGCCGAGAAACTCGGTCTGAATCCCGTAACCCTGGAACGCACGGTGGCCGAATTCAACGCCGCAT
GCGTGCCCGGCGAATTCGGCGGCCAAGATCTCGACGACCTCCACACCGAGGGAATCGAACCAAAGAAATCCAACTGGGCC
CGACCGATTATTGTGCCCCCGTTCAGCGCCTATCCTCTCCGGCCCGGGATCACCTTCACCTATCTCGGCGTCAAGGTAGA
CAGCCGTGCGCGGGTCATCATGGAGACAGGTGAGCCGACAAAAAACCTGTTTGCTTCGGGGGAAATAATGGCGGGCAGCA
TTCTCGGCCAAGGTTATCTCGCTGGATTTGGAATGGCGATTGGTACCGTATTCGGCCGCATCGCGGGTTGGGAGGCCGCA
CGTCATGCAGGATTTTGATCTCGTAAAAATGCTGTCTGACTTGCCGTCGGCGCCGGAGCTGGAAGCCAGGCGCGTTATGG
AGGTGTGCAACGCGTGCCGCTATTGCGAAGGGTTCTGCGCGGTATTTCCTGCAATGACCTTGCAGCGTCATTTCGCCAGC
GGCGATCTCAGCCACCTCGCCAATCTCTGCCACTCGTGCCAAGGTTGCTATTACGCCTGCCAATACGCCCCTCCGCATGA
GTTCGGAATAAACGTTCCAAAGGCGCTGTCGGAGTTGCGGCTCGAGAGCTACGAGCAGCATGCTTGGCCCCGGCCGGTCG
CCGCTCTCTATCGCAAGAATGCGCTCATCATTTCCATCTTGTCGGCGGCATGCATAACCGGCGTCCTTCTGCTTGCCGCC
ATCTTCAACGGGGATGCACTTTTCGCGAAACACGCATCGGTGCCCGGCGGCGGGTTTTACAACGTTATTCCTTATCAGGC
GATGATTGCCGTCGCGGCGACCACATTTCTTTATTCCGCGCTGGCGCTGGCGATCAGTCTCGTTCGCTTTTCGCGGACGA
TCGGTCTGGGAATTAAGGTTCTTTATCAGCACGTGCCGGTTCTTCGGGCGCTACGCGATGCGGCGACTCTGCGATATCTC
GGCGGCAGCGACGGCGAGGGGTGTAACGACGCGGACGAGACATTTTCGACGACCCGGCGAAAATTTCATCACGCCCTTGC
CTATGGCTTCGGACTTTGTTTCGCGGCCACAGCCACGGGCACGATCTACGATCATATGTTCGGCTGGCCGGCGCCCTATG
CGCTTTTCAGCTTGCCGGTCGTCCTAGGGACCGTTGGGGGGATCGGAATGGTCGTGGGCGCGATCGGCCTACTCTGGCTC
AAGCTGGCCGGCGAAGACGCTCCTCGATCACCGGCACTGCTTGGGCCGGATGTTGCCCTGTTGGTGCTTCTGCTTGCCAT
AGCGGCAACGGGCCTCCTCCTTTTAGCGGTCCGCAGCACCGAAGTCATGGGCGTCGCGCTCGCCGTCCATCTCGGCGTCG
TCTTGGCCTTCTTTTTGGTGATGCCATACAGCAAATTTGTCCACGGTATCTTCAGGCTCACGGCTCTCGTGCGCCATCAT
GCTGACCGCGAGGCAAGTAATGGCTTCGCCTCCAGCCCTCCCACGAAAAAGGGTTAAACAATGGAACATATGAAGTCCGT
TCGCGATCGCAGTAGCGTCATGCAGATCGTGAGAGTGGCGAGTGGCAACTGTCTCGAGCAATATGATTTCTTCGTTTACG
GCTTCTATGCGGCATATATTGCGAGAAGCTTTTTTCCGACCGGCGATAACGCGACATCGCTCATGCTTTCATTGGCCACT
TTTGGCGCTGGTTTCCTCATGAGGCCCTTGGGGGCGATTTTTCTCGGGTCCTACATCGATCGCGTCGGGCGTCGGAAAGG
CCTGATCGTGACACTCGCGATCATGGCCGTCGGAACCCTCACCATTGCGATGACTCCAAGCTATGAGGCAATTGGATTAC
TCGCACCGGTTATCGTGCTCGTCGGGCGACTTTTGCAGGGTTTTTCCGCTGGAGCAGAGTCGGGTGGCGTCTCAGTGTAC
TTGGCGGAAATTGCGTCGCCCAAATCGAGAGGCTTCTTCACCTCGTGGCAGTCTGCCAGCCAGCAGGTGGCCGTCATGAT
CGCCGCCGCGATCGGTCTTGCGCTGCAATCAACGCTTTCACCGGAGCAAATGAACGACTGGGGATGGCGGGTGCCCTTGT
TGATCGGATGCTTGATTATCCCCGTGATACTCTGGCTGCGCCGGTCTCTCCCGGAAACGAAAGCCTATCTCCACATGGAG
CACAAGGCGCATTCGATCGGCGAATCCCTCCGCGAATTGCAACAGAGCTGGGGGCTGATCTTGACGGGCATGGCGATGTC
GATCCTCACGACGACCACCTTTTACATGATTACCGCCTATACGCCGACATTTGGCGAGAAAGCACTCGGACTGAGCCCGC
AAGATGTCCTGCTGGTTACCATCATGGTCGGCGTGTCGAACTTCCTGTGGCTTCCGATCGGGGGTGCTCTCTCGGATCGT
ATCGGTAGAACCCCGATCCTACTGGTCGTGCCGGTCACCGTTCTCGCCATCGCCTTTCCCCTGATGAGCTGGCTCGTCGC
GGCACCGACATTCGGAGCGCTTGCAGCTGTTCTGCTGACTTTCTCCGCATGCTTTGGACTCTATAATGGGGCGCTCATCG
CGAGACTCACCGAGATTATGCCTCCCGCCATTAGAACCCTTGGCTTCTCGCTGGCGTTCAGTCTCGCGACCTCGCTGTTC
GGCGGCTTCACCCCATTGGTAAGTACGGCGCTAATCCACGCGACGGGCAGCAATTCCGCGCCTGCAATCTGGCTCTGTTT
TGCGGCTTTCATCAGCTTCGTCGGTGTGGCCGCATCGACCCGGCTGAGCCGGCCAATCGCCGAAGGCGCCAGATAGGACA
ATCAGAGAATGCCCGTGCGGCAATGAAGCGAGATTCGGGCGGTAGGTGCGCTGGCGGCACTTCGCGAAGAGCCGTTGCGG
ACGGCTGAAACGATGATGGTATGAATGGGCTAAGACATGAGAGCAGTAGTTTACCGAAATGGCGAACTTGTCCTGGGGGC
CTATGCTGATCCGATACCCGCCGCCGGGCAGGTGCTCGTCAAGACCAGAGCATGCGGCATCTGCGGATCTGACCTTCATT
TTTGCGATCATGCGCAGGCGTTTACGAACCTTGCATCGCGGGCGGGTATCGCCTCTATGGAAGTTGATTTGTGTCGAGAC
ATCGTTCTGGGGCATGAATTCTGTGGCGAGATTATGGAGTTCGGGCCCTCTGCGGATCGTCGCTTCAAACCCGGACAGCT
TGTGTGCTCGCTGCCGCTGGCGATCGGTCCGACCGGAGCGCGGACGATTGGCTACTCGGATGAGTATCCCGGCGGGCTCG
GCGAATATATGGTCCTCACGGAAGCGCTCTTGCTGCCTGTTCCGAACGGCCTTCCGGCGACCTGCGCGGCGTTGACGGAG
CCGATGGCGGTGGGATGGCATGCCGTCGAGATCGCGCAGGTTCAACCACATCACATCCCTGTGGTGATCGGGTGCGGACC
GGTCGGGTTGGCAGTCGTCGCTGCCCTGAAACATAAGCAAGTTGCTCCGATTATTGCGTCGGATCCATCGCCCGATCGGC
GTGCTCTTGCTCTGCGGATGGCGCCGACGCCGTTGTCGATCCGCGCGAAGAATCACCCTTTCGCCAGGCCGAGAAGATC
GCACGCCCGGTCGGACAAGGTGGGGCCCTGTCCAGCTCATTGCTGTCAAAGTCTCAAATGATATTCGAATGCGTAGGGGT
GCCGGGCATGCTTCGGCATGCGATGGACGGCGCGTCCGACGGGTCCGAGATCATGGTCGTTGGCGCATGCATGCAGCCGG
ACGCGATCGAGCCCATGATCGGGATGTTTAAAGCGCTCACGATCAAATTCTCGCGAACTTACACGGGTGAGGAATTCGCC
GCGGTGCTTCACATGATAGGTGAGGGCGCACTCGACGTATCTCCGCTCGTTACCGATGTGATTGGCCTGTCCGATGTCCC
 
GTCCGCGTTTGAGGCTCTACGGAGTCCAGGCGCCCAAGCAAAAGTGATTGTGGACCCTTGGCGCTGAGCCTGAGGATGCC
AAGGGTGCGACGTTGGGCATCGTCAAAGAAGGCGACGTTGACCCGGTATGTGAACATCCCCATATTCTTCCGCAGCTGAA
GCAGTTGGTAAACATGCCAAAATATGAACTGTAGTATTGCGTCGGGGTTCTCATTGTGGGGTTTGCCATTGTCATCGCTC
GCACCCGGCGACAAAGATTAGATGTACTTCCGATAATCCGTGCTCTCGACCTGGCCTTCCTTCATATATTTCAGGACCTC
TCCGACCATGCGTGCGGCGCGGATCGGGATCGGCAGGCGTTGGTTCATCTGGGTCGAGTTCCAGTTGATCTTCGTAAGAG
AGAACACCTCCTCGGCTAACTGCGCCGCGGTACTATCGCAGGATCGTCTCGAGCGTYCGC
>Seq ID 8 (*fumD*) 
GTGAAAGAGCACCAATGCCGTGGCGGCCGGGCGTCCCCCGCTGCGCCCGCCACGTGGCTTGCGCGGATCAGCGTTTCCCG
GGGGGCCTCCGCCATCGCCTGGACCTTCATGCTTGGCGCAACTGCCATTCCCGTGGCTGCGCAAACTGACGATCCGAAGC
TCGTTCGTCATACCCAGTCGGGCGCCGTCGAGGGCGTCGAGGGCGACGTCGAGACTTTTTTGGGAATACCCTTCGCGGCT
CCGCCGGTCGGCGACCTGCGATGGCGGCCGCCGGCTCCGCCGAGGGCGTGGGCGGCACCAGGGACGGCCGCCGCTTTGC
GCCCGATTGCATCGGGAACGAGCGGCTTAGAGAGGGGAGCCGGGCTGCCGGGACGAGCGAAGACTGCCTCTATCTGAATA
TCTGGTCTCCCAAACAGGTCGGTAAGGGGGGGCTCCCCGTCATGATCTGGGTTTACGGCGGTGGGTTTAGCGGCGGTTCT
GGCGCGGTGCCATATTATGACGGCTCTGCGCTCGCGCAGAAGGGCGTGGTGGTCGTCACGTTCAACTATCGCGCCGGGAT
TCTGGGCTTTCTTGCCCATCCGGCGCTTTCAAAGGAAAGTCCGAATGGCGTGTCGGGCAACTATGGTCTTCTCGACATGC
TCGCGGCGTTCAAATGGGTTCAGAACAACATAAGGGAGTTCGGCGGAGACCCGAACCGTGTCACGGTCTTTGGCGAGTCC
GCCGGCGCGAGCGCGCTCGGACTGCTCCTGACCTCGCCGCTCAGTGAGAGCGCCTTCAATCAGGCGATACTGCAAAGTCC
GGGTCTGGCCAGGCCGCTCGCCACGCTTTCTGAAAGCGAAGCGAATGGGCTGGAGCTGGGAGCCGATATTTCTGCTCTAC
GGCGTGCCGATGCGGGCGAATTGACGAAGATCGCGCAATCGCGAATACCCATGTCGCGCCAGTTCACCAAGCCGCGGCCG
ATGGGTCCGATTCTGGACGGCTATGTTTTGCGCACCCTTGACGTCGATGCCTTCGCCAAGGGGGCCTTCCGCAAGATACC
CGTTCTGGTCGGCGGAAACGCCGACGAAGGGCGCGCTTTTACGGATCGCCTGCCGGTCAAAACGGTCCTTGAATATCGAG
CCTATCTCACAGAACAATTTGGTGACGAGGCGGACGCATGGAGCGTTGTTATCCCGCGAACTCCGACGCCGACGTCCCC
GCCGCCGTTGCCCGTCTTTTTGGGGATAGTCAGTTCAACAACGGGATCGAGCTGCTCTCGGCAGCCTTCGCGAAATGGCG
AACGCCGCTTTGGAGATATCGCTTTACGGGCATTCCAGGAGCCGGCCGTCGCCCCGCCACGCATGGAGACGAAATTCCCT
ATGTCTTCGCAAATCTGGGGCCGTCGTCCGTATCTATGTTTGGGTCGCTCGAAGGCGGCGCCGGGGCGTCGGACATCAAA
CTTGCGACCGAAATGTCCGCGGCCTGGGTGAGCTTCGCGGTGCACGGGGTCCCCGATCAGGGCACGAAATCGCACTGGCC
GCGCTTCGAGCGGCGAGGGGAGATCATGACTTTTGGTTCGCAGGTTGGCTCTGGGGAAGGTCTTGGAGTTTCGCCGAGCA
AAGCCTGCCAACCCTCAAAATAG
>Seq ID 18 (*fumI*)

ATGGCGAACGGAACAAGGCAGAAAGATCTCAGAGAACGCGCCGAACGGGTCATTCCGGGCGGATGTACGGCCACGAG
TCGACACGGTTGCTGCCGCCAGAATTCCCCCAGTTCTTCAGGCGCGCGCTGGGGGCACGAATTTGGGACGCCGACGAG
CAGCCCTATATCGACTATATGTGCGCGTATGGGCCAAATTTGCTCGGTTACCGGCAATCCGAAATCGAAGCCGCGGCT
GATGCGCAGCGACTTCTCGGCGACACCATGACCGGTCCTTCGGAGATCATGGTCAACCTCGCCGAAGCCTTTGTGGGC
ATGGTCCGTCATGCGGATTGGGCGATGTTCTGCAAAAATGGCAGCGATGCCACCTCAACGGCGATGGTTCTCGCGCGT
GCCCATACGGGGCGCAAAACCATATTATGCGCCAAAGGCGCCTATCATGGCGCTTCCCCGTGGAACACTCCGCATACT
GCCGGGATTCTCGCTTCCGATCGCGTGCATGTCGCATATTATACCTATAACGACGCCCAAAGCTTATCGGACGCGTTC
AAGGCGCACGATGGCGATATTGCGGCTGTCTTTGCCACACCTTTCCGACACGAAGTATTTGAGGACCAGGCCCTCGCC
CAGCTTGAGTTCGCGCGCACCGCTCGAAAATGTTGTGACGAGACCGGTGCGCTTCTGGTCGTTGACGATGTGCGCGCA
GGTTTCCGGGTGGCGCGCGATTGCAGCTGGACGCATTTGGGTATCGAACCCGATCTCAGTTGCTGGGAAAATGCTTT
GCGAATGGCTATCCGATCTCCGCCCTGCTGGGCTCGAACAAGGCGCGCGATGCGGCGCGGGATATATTTGTGACCGGC
TCCTTCTGGTTCTCTGCGGTACCGATGGCGGCCGCGATCGAAACCCTCAGGATCATTCGAGAGACGCCTTATCTCGAA
ACGCTGATCGCCAGCGGCGCCGCCCTGCGGGCAGGCCTGGAGGCACAGTCTCAGCGCCATGGTCTTGAGTTGAAGCAG
ACGGGCCCGGCGCAGATGCCGCAAATATTCTTTGCGGACGATCCCGATTTTCGGATCGGCTATGCGTGGGCCGCGGCG
TGCCTGAAGGGCGGCGTCTATGTTCATCCCTATCACAATATGTTTCTCTCTGCGGCCCATACAGTTGACGATGTAACG
GAGACCCTCGAGGCGACGGATCGCGCGTTCAGCGCGGTCCTCAGAGATTTTGCGTCTCTCCAGCCTCATCCCATTTTA
ATGCAACTCGCCGGTGCTTGA

### Enzyme

### Sequenzen:

>Seq ID 9 (FumD) 
VKEHQCRGGRASPAAPATWLARISVSRGASAIAWTFMLGATAIPVAAQTDDPKLVRHTQS
GAVEGVEGDVETFLGIPFAAPPVGDLRWRPPAPPRAWAGTRDGRRFAPDCIGNERLREGS
RAAGTSEDCLYLNIWSPKQVGKGGLPVMIWVYGGGFSGGSGAVPYYDGSALAQKGVVVVT
FNYRAGILGFLAHPALSKESPNGVSGNYGLLDMLAAFKWVQNNIREFGGDPNRVTVFGES
AGASALGLLLTSPLSESAFNQAILQSPGLARPLATLSESEANGLELGADISALRRADAGE
LTKIAQSRIPMSRQFTKPRPMGPILDGYVLRTLDVDAFAKGAFRKIPVLVGGNADEGRAF
TDRLPVKTVLEYRAYLTEQFGDEADAWERCYPANSDADVPAAVARLFGDSQFNNGIELLS
AAFAKWRTPLWRYRFTGIPGAGRRPATHGDEIPYVFANLGPSSVSMFGSLEGGAGASDIK
LATEMSAAWVSFAVHGVPDQGTKSHWPRFERRGEIMTFGSQVGSGEGLGVSPSKACQPSK
>Seq ID 19 (FumI)

MANGTRQKDLRERAERVIPGGMYGHESTRLLPPEFPQFFRRALGARIWDADEQPYIDYMC
AYGPNLLGYRQSEIEAAADAQRLLGDTMTGPSEIMVNLAEAFVGMVRHADWAMFCKNGSD
ATSTAMVLARAHTGRKTILCAKGAYHGASPWNTPHTAGILASDRVHVAYYTYNDAQSLSD
AFKAHDGDIAAVFATPFRHEVFEDQALAQLEFARTARKCCDETGALLVVDDVRAGFRVAR
DCSWTHLGIEPDLSCWGKCFANGYPISALLGSNKARDAARDIFVTGSFWFSAVPMAAAIE
TLRIIRETPYLETLIASGAALRAGLEAQSQRHGLELKQTGPAQMPQIFFADDPDFRIGYA
WAAACLKGGVYVHPYHNMFLSAAHTVDDVTETLEATDRAFSAVLRDFASLQPHPILMQLA
GA

Zur Lösung dieser Aufgaben ist ein derartiger Zusatzstoff dadurch gekennzeichnet, dass ein Enzym der Sequenz ID-Nr. 9 sowie gegebenenfalls zusätzlich ein Cosubstrat für das eingesetzte Enzym, ein Enzym der Sequenz ID-Nr. 19 und ein inerter Träger enthalten sind.

Ein derartiger Zusatzstoff, in dem Enzym oder ein kompletter, rekombinanter Wirtsorganismus zur Expression dieses Enzyms sowie gegebenenfalls zusätzlich ein Cosubstrat für das eingesetzte Enzym, ein Enzym der Sequenz ID-Nr.19 und ein inerter Träger enthalten sind, zeichnet sich dadurch aus, dass er zielgerichtet Fumonisine, abbaut und somit detoxifiziert. Durch den Einsatz eines erfindungsgemäßen Zusatzstoffes, welcher im wesentlichen aus einem isolierten Enzym sowie gegebenenfalls dessen Cosubstrat und Trägern besteht, ergibt sich der Vorteil, dass diese ihre katalytische Aktivität in einer Umgebung und unter Bedingungen beibehalten, in welchem beispielsweise komplette Mikroorganismen nicht oder nur wenig aktiv wären, wobei gleichzeitig eine bedeutend höhere, spezifische Aktivität erzielt werden kann, sowie definierte Reaktionen unter Vermeidung von unerwünschten Nebenreaktionen katalysiert werden können.

Darüber hinaus können Probleme, welche gemäß dem Stand der Technik durch den Einsatz vermehrungsfähiger Keime auf landwirtschaftlichen Rohprodukten entstanden sind, mit Sicherheit hintangehalten werden und überdies weisen Zusatzstoffe, welche nur isolierte Enzyme enthalten, sowohl eine bessere Eignung zur Formulierung für eine gezielte und kontrollierte Aktivierung, d.h. beispielsweise an einer bestimmten Stelle des Verdauungstrakts, als auch die Vermeidung von unerwünschtem, erhöhtem Substratverbrauch auf.

Weiterhin kann der Sphingolipidstoffwechsel, der durch eine Wechselwirkung der Fumonisine mit dem Enzym Ceramidsynthase behindert wird, aufrecht erhalten werden und gleichzeitig die Fumonisine biologisch zu nicht toxischen Substanzen abgebaut werden. Schließlich können technologische Anwendungen der Detoxifizierung erzielt werden.

Mit einem derartigen Zusatzstoff gelingt es, einerseits beispielsweise Mykotoxine direkt auf dem Rohmaterial vollständig und zuverlässig abzubauen, wobei die spezifischen bei diesem Verfahren produzierten Enzyme den Abbau von Fumonisinen und von Zwischenprodukten des Abbauweges katalysieren, und andererseits Mykotoxine beispielsweise auch direkt bei der Bioethanolherstellung in der Maische für die Alkoholherstellung abzubauen oder auch bei der Herstellung von Nahrungsmitteln direkt in dem Herstellverfahren abzubauen bzw. unschädlich zu machen.

Indem der Zusatzstoff so ausgebildet ist, wie dies einer bevorzugten Weiterbildung der Erfindung entspricht, dass das eine Enzym mit einer Schutzhülle ummantelt eingesetzt ist, kann sichergestellt werden, dass das Enzym vor einem vorzeitigen Aktivitätsverlust gesichert ist und sicher und zuverlässig an der vorgesehenen Stelle beispielsweise im Magen-Darm-Trakt seine Wirkung entfaltet.

Durch Verkapseln des Enzyms in einer Schutzhülle gelingt es, das Enzym ohne Veränderung, insbesondere ohne Abbau und Schädigung an ihren Einsatzort, insbesondere beispielsweise in den Verdauungstrakt, zu transportieren, so dass erst nach Auflösung der Schutzhülle, beispielsweise im Magen-Darm-Trakt von Menschen oder Tieren, das Enzym bzw. gegebenenfalls die Enzyme zu wirken beginnt(en), wodurch ein noch gezielterer, rascherer und vollständiger Abbau der Mykotoxine im sauerstoffunabhängigen Milieu des Magen-Darm-Trakts erzielt werden kann, und gleichzeitig Fumonisine daran gehindert werden können, ihre schädliche Wirkung auf diejenigen Lebewesen auszuüben, welche diese mit der Nahrung aufgenommen haben.

Indem der Zusatzstoff so ausgebildet ist, dass das Enzym eine Carboxylesterase Sequenz ID-Nr. 9 ist, wird im Wesentlichen das zum Substratkatabolismus befähigte Enzym zum Einsatz gebracht, so dass neben einer geringeren Menge an einzusetzendem Enzym auch sichergestellt werden kann, dass keine unerwünschten Nebenreaktionen bei Einsatz des Enzyms auftreten.

Gemäß einer bevorzugten Weiterbildung der Erfindung ist der Zusatzstoff so ausgebildet, dass eine Carboxylesterase Sequenz ID-Nr. 9, eine Aminotransferase Sequenz ID-Nr. 19, eine α-Ketosäure als Cosubstrat und ein inerter Träger enthalten sind. Indem der Zusatzstoff eine Carboxylesterase, eine Aminotransferase und eine α-Ketosäure als Cosubstrat neben einem inerten Träger aufweist, gelingt es insbesondere, Fumonisine, welche in Nahrungsmitteln enthalten sind, zuerst zu hydrolysieren, indem von den Fumonisinen Tricarballylsäurereste mit Hilfe einer Carboxylesterase abgespalten werden, und das so gebildete hydrolysierte Fumonisin in weiterer Folge unter Einwirkung der Aminotransferase und der α-Ketosäure als Cosubstrat, im vorliegenden Fall bevorzugt Brenztraubensäure, weiter umzusetzen, indem eine Aminogruppe von dem hydrolysierten Fumonisinmolekül durch eine Ketogruppe ersetzt wird, so dass ein für beispielsweise Säuger völlig ungefährliches 2-Keto-hydrolysiertes Fumonisin entsteht, welches unverändert ausgeschieden werden kann, und als Nebenprodukt Alanin gebildet wird, welches vollständig unschädlich ist und ebenfalls keinerlei negative Eigenschaften beispielsweise auf einen Organismus ausübt bzw. aufweist, so dass ein vollständiger Abbau von Fumonisinen zu unschädlichen Substanzen sichergestellt ist.

Gemäß einer bevorzugten Weiterbildung der Erfindung ist der Zusatzstoff derart weitergebildet, dass eine Carboxylesterase Sequenz ID-Nr. 9, wenigstens ein Adsorbens, wie ein Tonmineral, sowie ein inerter Träger enthalten sind. Bei Einsatz von lediglich einer Carboxylesterase Sequenz ID-Nr. 9 und wenigstens eines Adsorbens kann die Detoxifizierung der Fumonisine auch so geführt werden, dass lediglich die Tricarballylsäurereste abgespalten werden und das so gebildete, hydrolysierte Fumonisin an dem Adsorbens adsorbiert wird. Indem die Tricarballylsäurereste durch den Einsatz der Carboxylesterase abgespalten werden, entsteht ein im Wesentlichen langkettiges Molekül, welches leicht und zuverlässig adsorbiert werden kann, so dass lediglich durch einen gezielten Einsatz eines einzigen Enzyms eine vollständige Detoxifizierung durch insbesondere sauerstoffunabhängigen Abbau des Fumonisins und anschließende Adsorption sichergestellt werden kann.

Indem bei Einsatz von Carboxylesterase Sequenz ID-Nr. 9 zusätzlich wenigstens ein Adsorbens, gewählt aus Tonmineralien, eingesetzt wird, kann auch ohne Zusatz von weiteren Enzymen ein vollständiges Unschädlichmachen der Fumonisine erzielt werden, indem in einem ersten Schritt durch die Carboxylesterase aus dem Fumonisinmolekül die zwei Tricarballylsäure-Seitenketten abgespalten werden und sogenanntes hydrolysiertes Fumonisin gebildet wird. Hydrolysiertes Fumonisin, welches ein im wesentlichen kettenförmiges Molekül ist, kann in weiterer Folge beispielsweise an Tonerdemineralien adsorbiert werden, so dass auch in einem einstufigen, enzymatischen Abbauverfahren ein vollständiges Unschädlichmachen der Fumonisine, erzielt werden kann.

Indem hierbei Fumonisine, sauerstoffunabhängig bzw. anaerob abgebaut werden, gelingt es, dass die Nukleinsäuresequenzen von Genen bzw. Enzymen ohne Zusatz von molekularem Sauerstoff sicher und zuverlässig die Abbaureaktionen durchführen, wodurch der so hergestellte Zusatzstoff in sämtlichen sauerstoffunabhängigen bzw. anaeroben Medien, wo Mykotoxine möglicherweise abgebaut werden müssen, zum Einsatz gelangen kann, wie beispielsweise in Nahrungsmitteln für Menschen und Tiere, der Bioethanolherstellung, aber auch zur Herstellung bzw. Produktion von gentechnisch veränderten, landwirtschaftlichen Nutzpflanzen.

Indem hierbei die Enzyme aus der Carboxylesterase Sequenz ID-Nr. 9 und gegebenenfalls der Aminotransferase Sequenz ID-Nr. 19 gewählt werden, können Fumonisine glatt und vollständig im sauerstoffunabhängigen Milieu abgebaut werden. In diesem Fall wird bei den aus dem Gencluster der Nukleinsäuresequenz mit der Sequenz ID-Nr. 1, welche aus einem prokaryotischen Stamm mit der Hinterlegungsnummer DSM 16254 stammt, isolierten Fum-Genclustern die Transkription der offenen Leserahmen durch einen bidirektionalen Promotor gesteuert bzw. geregelt, der zwischen FumA und FumI, wie dies der nachfolgenden Tabelle 1 entnehmbar ist, angeordnet ist. Die Cluster codieren für Proteine, welche in der Regulierung der Genexpression, bei der Substratabtastung, dem Transport und in dem Substratkatabolismus, wie beispielswiese FumD oder FumI involviert sind. Aus diesen Nukleinsäuresequenzen, welche für spezielle Gene bzw. Enzyme codieren, wurden die Gene ausgewählt, welche für den Substratkatabolismus verantwortlich sind, wodurch die entsprechenden gebildeten Enzyme das Substrat, nämlich Fumonisine vollständig katabolisieren können.

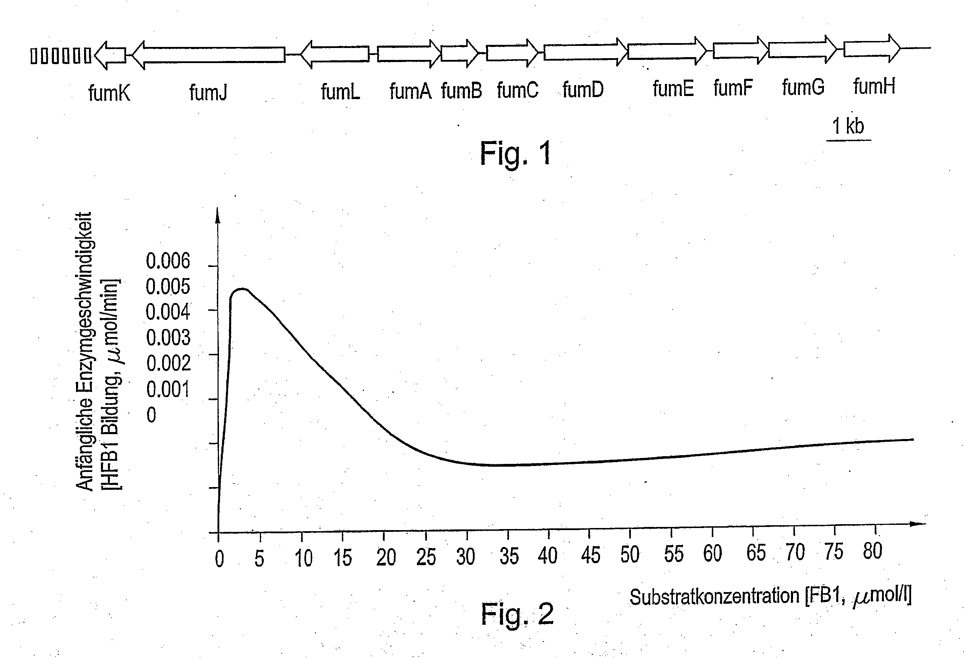

In diesem Fall werden beispielsweise aus dem Gencluster der Nukleinsäuresequenz mit der Sequenz ID-Nr. 1 ausgewählte, offene Leserahmen in prokaryotischen oder eukaryotischen Wirtszellen zur Expression gebracht. Im bakteriellen Stamm mit der Hinterlegungsnummer DSM 16254 erfolgt die Transkription der im Gencluster mit der Sequenz ID-Nr. 1 enthaltenen, offenen Leserahmen, gesteuert bzw. geregelt durch einen bidirektionalen Promotor, der zwischen fumA und fumI, wie dies der nachfolgenden Fig. 1 entnehmbar ist, angeordnet ist. Die Gene codieren für Proteine, welche in der Regulierung der Genexpression, wie beispielsweise bei der Substraterkennung, dem Transport und im Substratkatabolismus, wie beispielsweise FumD und FumI involviert sind.

In der nachfolgenden Tabelle 1 ist die Bezeichnung der Gene des fumonisinkatabolischen Genclusters aufgeführt, wobei O die Orientierung, nämlich f forward und r reverse, bedeuten.

**Tabelle 1**

| Gen | Sequenz ID | O | Start | Ende | Länge | Bezeichnung |
|---|---|---|---|---|---|---|
| *fumD* | 8 | f | 8294 | 9916 | 1623 | Carboxylesterase |
| *fumI* | 18 | r | 5063 | 3795 | 1269 | Aminotransferase |

Gemäß einer Weiterbildung der Erfindung wird eine Verwendung des Zusatzstoffes in einem sauerstoffunabhängigen Milieu bei der Bioethanolherstellung, gemeinsam mit einer Maische bzw. einem pflanzlichen Ausgangsmaterial, durchgeführt, wobei der Zusatzstoff so gewählt ist, dass das darin enthaltene Enzym vollständig aus Bakterien stammt, die den Katabolismus von Fumonisinen über einen hochspezifischen Abbauweg katalysieren, wodurch es gelingt, sie mit hoher Spezifität, Aktivität und Effizienz einzusetzen, wodurch der Zusatzstoff auch in einem sauerstoffunabhängigen Milieu technologisch verwendet werden kann.

Schließlich ist eine Verwendung von Genen, wie sie in den Sequenzen ID-Nr. 8 und 18 dargestellt sind, oder einem Cosubstrat, einer Carboxylesterase Sequenz ID-Nr. 9 gegebenenfalls, einer Aminotransferase Sequenz ID-Nr. 19, sowie einer α-Ketosäure als Cosubstrat zur Herstellung eines Zusatzstoffes für den Abbau von Fumonisinen, bei der Verarbeitung oder Verwendung von pflanzlichen Rohstoffen möglich. Mit einem derart hergestellten Zusatzstoff gelingt ein vollständiger und zuverlässiger Abbau von Fumonisinen, insbesondere in einem sauerstoffunabhängigen Milieu. Insbesondere gelingt es mit einer derartigen Verwendung Fumonisine sicher und zuverlässig beispielsweise in pflanzlichen Rohstoffen bzw. Ausgangsmaterialien zur Gänze zu unschädlichen Bestandteilen abzubauen.

Eine weitere Verwendung ist der Einsatz einer Carboxylesterase, wenigstens eines Adsorbens, wie eines Tonminerals, sowie eines inerten Trägers. Durch Verwendung einer Carboxylesterase Sequenz ID-NR. 9 und wenigstens eines Adsorbens gelingt es, Fumonisine sicher und zuverlässig durch lediglich den Einsatz eines einzigen Enzyms zu entgiften, indem mit diesem Enzym bzw. mit Hilfe dieses Enzyms die Tricarballylsäure-Seitenreste von dem Fumonisin abgespalten werden und das dabei gebildete, langkettige, hydrolysierte Fumonisin in der Folge an dem Adsorbens adsorbiert wird, wodurch das Toxin sicher und zuverlässig unschädlich gemacht wurde.

Gemäß einer weiteren Verwendung wird der Zusatzstoff gemäß der Erfindung zur sauerstoffunabhängigen bzw. anaeroben Behandlung eines pflanzlichen Ausgangsmaterials bzw. einer Maische in der Bioethanolherstellung eingesetzt. In diesem Fall gelingt es, die in dem pflanzlichen Ausgangsmaterial bzw. Rohstoff enthaltenen Mykotoxine während der Bioethanolherstellung im sauerstoffunabhängigen Milieu sicher und zuverlässig unschädlich zu machen, so dass der Rückstand aus der Ethanolproduktion, nämlich der Trester oder die Trockenschlempe, nachfolgend entweder direkt oder nach Trocknung und Pelletierung ohne weitere Verarbeitung, insbesondere Detoxifizierung, als Futtermittel, welches frei von Fumonisinen ist, eingesetzt werden kann.

Indem der Zusatzstoff in einem zu vergärenden, pflanzlichen Ausgangsmaterial bzw. in einer Maische zur Bioethanolherstellung eingesetzt wird, gelingt es, dass bei der Ethanolherstellung anfallende Coprodukte, nämlich den Trester, d.h. die getrockneten Körnerreste und unlösliche Bestandteile oder die Trockenschlempe (Dried Distiller's Grains with Solubles - DDGS), von Fumonisinen bzw. Mykotoxinen insbesondere in einem sauerstoffunabhängigen Milieu zu befreien.

Die Erfindung wird nachfolgend anhand von Ausführungsbeispielen sowie Figuren näher dargestellt. In diesen zeigt:
Fig. 1 den Fumonisin-katabolischen Gencluster,
Fig. 2 die Michaelis-Menten-Kurve für Fumonisin-Carboxylesterase FumD,
Fig. 3 eine Abbaukurve von hydrolysiertem Fumonisin Bi,
Fig. 4 die Umwandlung von Fumonisin FB1 in hydrolysiertes Fumonisin HFB 1 nach Zugabe von Carboxylesterase ID-Nr. 9 zeigt, und
Fig. 5 den Abbau von hydrolysiertem Fumonisin HFB1 durch Zusatz von Aminotransferase ID-Nr. 19.

In Fig. 1 ist ein Fumonisin katabolischer Gencluster als Teilsequenz von 15420 Basenpaaren eines mikrobiellen Stammes mit der Hinterlegungsnummer DSM 16254 dargestellt. In dem fum-Gencluster des prokaryotischen Stammes DSM 16254 wird die Transkription der offenen Leserahmen durch einen bidirektionalen Promotor gesteuert bzw. geregelt, welcher zwischen fumA und *fumI* angeordnet ist. Der Cluster codiert Proteine, welche in der Regulierung der Genexpression, wie beispielsweise FumB und FumC, in der Substraterkennung und dem Transport, wie beispielsweise FumJ, FumA und FumG und in einem Substratkatabolismus, wie beispielsweise FumD, FumE, FumF, FumH, FumI und FumK involviert sind.

### Beispiele

### Beispiel 1: Die Enzymkinetik von Fumonisincarboxylesterase.

Das *fumD* Gen (Sequenz ID-Nr. 8), welches eine Fumonisincarboxylesterase codiert, wurde kloniert und in *Pichia pastoris* unter Verwendung von Standardverfahren exprimiert. Das his-getaggte Enzym wurde rückgewonnen und aus der überstehenden Lösung der Kultur durch Affinitätschromatographie gereinigt. Die Enzymkonzentration wurde bestimmt und die enzymkinetischen Parameter wurden mit sieben unterschiedlichen Substratkonzentrationen im Bereich zwischen 50 µg bis 25 mg FB₁ pro Liter und einer Enzymkonzentration von 0,33 ng/ml bestimmt. Die Reaktionen wurden in 20 mM Tris-Cl (pH 8,0) Puffer mit 0,1 mg/ml Rinderserumalbumin gepuffert und bei 30 °C inkubiert. Proben wurden nach 0, 30, 60 120 und 240 Minuten Inkubation genommen und durch HPLC-MS/MS analysiert. Fumonisin B₁ (FB₁) und hydrolysiertes Fumonisin B₁ wurden quantifiziert basierend auf einer Kalibrierung mit den gereinigten Referenzsubstanzen und einem vollständig ¹³C markierten, internen FB1 Standard.

Fig. 2 zeigt die Michaelis-Menten-Kurve für die Hydrolyse von Fumonisin B1 (FB1) durch Fumonisin-Carboxylesterase FumD, welcher bei einer Enzymkonzentration von 0,33 ng/ml in Tris-Cl-Puffer (pH 8,0) bestimmt wurde, wobei anfängliche Enzymgeschwindigkeiten gegen die Substratkonzentration aufgetragen wurden. Die Michaelis-Menten-Kurve zeigt einen Abfall bei höheren Substratkonzentrationen, da die Enzymgeschwindigkeit basierend auf dem Produkt, d.h. hydrolysierter FB₁ -Bildung berechnet wurde. Da hydrolysiertes FH₁ aus FB₁ in einer zweistufigen Reaktion über partiell hydrolysiertes FB₁ mit lediglich einer Tricarballylsäure-Seitenkette, die zurückbehalten wurde, und einer Seitenkette ausgebildet wird, die abgespalten wurde, war die Endproduktbildung bei hohen Substratkonzentrationen verzögert. Die Michaelis-Menten-Konstante K_{M} wurde als 0,90 µmol/l berechnet, was 650 ppb äquivalent ist, und die Umwandlungszahl war 900 pro Sekunde.

Aus Fig. 2 ergibt sich, dass Fumonisine mit der Carboxylesterase in den relevanten Konzentrationsbereichen rasch und vollständig hydrolysiert werden können.

### Beispiel 2: Die katalytische Aktivität von HFB1 (hydrolysiertes Fumonisin B1) Aminotransferase

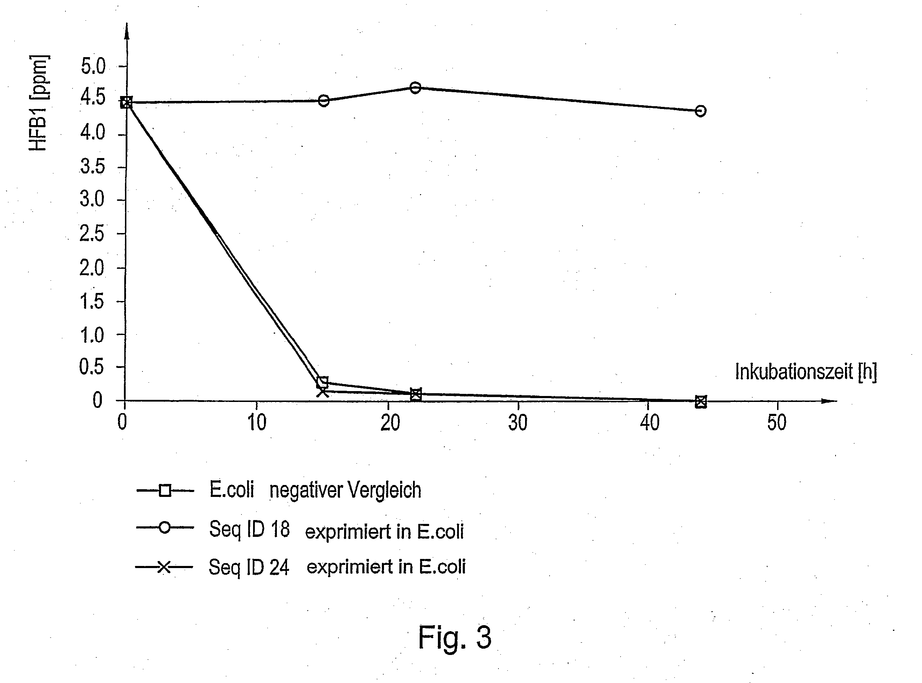

Sequenzen ID-Nr. 18 und 24 wurden unter Verwendung von Standardverfahren kloniert und in *E. coli* exprimiert unter Steuerung bzw. Regelung eines Bakteriophagen T7 Promotors. Die Bakterienzellen wurden gesammelt in 50 mM Natriumphosphatpuffer, neuerlich suspendiert und durch Ultraschall lysiert. Hydrolysiertes Fumonisin wurde hinzugefügt und die Proben wurden bei 25 °C inkubiert. Proben wurden in Zeitintervallen genommen und durch HPLC-MS/MS analysiert. Es wurde keine Reduktion der hydrolysierten FB₁ Konzentration beobachtet. Wenn ein Cosubstrat, wie beispielsweise eine α-Ketosäure, wie Brenztraubensäure oder Oxalacetat, zu der Reaktion hinzugefügt wurde, konnte ein vollständiger Abbau des hydrolysierten Fumonisins zu 2-Keto-HFB₁ beobachtet werden, wie dies in Fig. 3 dargestellt ist. Diese Substanz ist für Säuger vollständig unschädlich.

### Beispiel 3: Enzymaktivität in Darmmilieu

Zur Überprüfung der enzymatischen Aktivität von FUM-Carboxylesterase im Verdauungstrakt wurden schlachtfrische Schweinedärme verwendet und unter Ausschluss von Sauerstoff ins Labor transportiert und in einer anaeroben Sterilwerkbank untersucht. Etwa 10 cm lange Stücke von Duodenum und Jejunum wurden abgebunden und herausgeschnitten. Mit Kanülen wurde Fumonisin B1 in konzentrierter wässriger Lösung auf eine Endkonzentration von etwa 10 ppm verdünnt eingespritzt und mit Darminhalt vermischt. Anschließend wurden 5 µg Fumonisin-Carboxylesterase in wässriger Lösung, bzw. dasselbe Volumen Wasser in den Negativkontrollen, eingespritzt und eingemischt. Die Darmabschnitte wurden bei 39 °C inkubiert. Mit Hilfe von Kanülen wurden Proben gezogen und durch HPLC-MS/MS analysiert. Dabei zeigte sich, daß Fumonisin B1 im Duodenum und Jejunum zum Zeitpunkt der ersten Probenahme nach zwei Stunden bereits vollständig hydrolysiert war.

### Beispiel 4: Ermittlung des Temperaturbereichs der Aktivität von Fumonisin-Carboxylesterase

Zur Ermittlung des Temperaturbereichs, in dem Fumonisin-Carboxylesterase aktiv ist, wurden 1,6 ng/ml FUM-Carboxylesterase in 20 mM Tris-Cl Puffer, pH 7,0, mit 0,1 mg/ml BSA und 10 ppm Fumonisin B1 bei unterschiedlichen Temperaturen inkubiert. Dabei zeigte sich, dass das Temperaturoptimum für das Enzym bei 30 °C lag. Auch bei 40 °C und sogar 50 °C wurde enzymatische Aktivität noch eindeutig festgestellt. Somit ist diese FUM-Carboxylesterase zur Anwendung unter den Temperaturbedingungen wie im Verdauungstrakt, oder im Zuge von Prozessschritten der Lebens- oder Futtermittelherstellung, die bei erhöhter Temperatur stattfinden, geeignet.

### Beispiel 5: Bestimmung des pH-Bereichs der Aktivität von Fumonisin-Carboxylesterase

Zur Bestimmung des pH-Bereichs, in dem Fumonisin-Carboxylesterase aktiv ist, wurde Teorell-Stenhagen-Puffer verwendet. Dieser Puffer lässt sich durch die Kombination von Citrat, Phosphat und Borat über einen Bereich von 10 pH Einheiten mit gleicher Pufferkapazität einstellen. FUM-Carboxylesterase wurde in einer Konzentration von 3,3 ng/ml mit 10 ppm Fumonisin B1 bei verschiedenen pH-Werten in diesem Puffer bei 25 °C inkubiert. Die höchste Aktivität zeigte sich bei pH 8,0, aber es konnte im gesamten Bereich von pH 5 bis pH 10 Aktivität festgestellt werden. Durch die Aktivität in diesem breiten pH-Bereich wird die technologische Anwendung des Enzyms als Futtermittelzusatz bzw. im Zuge der Verarbeitung von Lebens- und Futtermitteln ermöglicht.

### Beispiel 6: Fütterungsversuch mit Ferkeln

Der Versuch wurde in einem Versuchsstall mit 12 Buchten für jeweils 10 Tiere durchgeführt. Der Stall war ausgestattet mit Spaltenboden, Schalentränkern und einem computergesteuerten Fütterungssystem. Die Automaten waren entlang der Buchtenwände angeordnet. Das Stallklima wurde täglich automatisch aufgezeichnet und die Temperatur nach den Standardempfehlungen zur Ferkelaufzucht eingestellt.

120 gemischt-geschlechtliche Absetzferkel (Alter: ca. 4 Wochen, durchschnittliches Einstellgewicht 8,21 kg) wurden für diesen Versuch eingesetzt. Jedes Ferkel wurde mit einer Ohrmarke versehen und einzeln gewogen. Die 120 Ferkel wurden auf 12 Buchten zufällig verteilt. Alle Ferkel entstammten dem österreichischen Zuchtprogramm ÖHYB (= (Edelschwein x Landrasse) x Pietrain).

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte 2-phasig: Absetzphase Tag 1 - 14, Aufzuchtphase Tag 15 - 42. Das Versuchsfutter wurde buchtenindividuell über die Spotmix-Fütterungsanlage gemischt und je nach Anzahl der Ferkel, Gewichtsentwicklung und Futterverzehr zweimal täglich trocken zugeteilt. Wasser stand zur Aufnahme ad libitum zur Verfügung. Die 12 Buchten wurden in vier verschiedene Applikationsgruppen zu je drei Wiederholungen geteilt und erhielten die folgenden Einmischungen ins oben beschriebene Futter:

| Gruppe | |
|---|---|
| Negativ-Kontrolle | Keine Toxine, kein Enzymzusatz |
| Positiv-Kontrolle | 4 - 5,5 ppm Fumonisin B1 |
| Versuchsgruppe 1 | 4 - 5, 5 ppm Fumonisin B1 + Enzymmix 1 (Carboxylesterase, Aminotransferase, Pyruvat) 0,5 kg/t Futter |
| Versuchsgruppe 2 | 4 - 5,5 ppm Fumonisin B1 + Enzymmix 1 (Carboxylesterase, Aminotransferase, Pyruvat, inerter Carrier) 1 kg/t Futter |

In der Positiv-Kontrolle wurden bei fast der Hälfte der Tiere respiratorische Probleme beobachtet, wobei es auch zu einem Ausfall kam. Alle anderen Gruppen erschienen gesund.

### Leistungsdaten

| Gruppe | Anzahl der Tiere | Anfangsgewicht (Durchschnitt, kg) | Endgewicht (Durchschnitt, kg) | Ausfälle |
|---|---|---|---|---|
| Negativ-Kontrolle | 30 | 8,34 | 26,82 | |
| Positiv-Kontrolle | 30 | 8,17 | 24,77 | 1 |
| Versuchsgruppe 1 | 30 | 8,08 | 26,69 | |
| Versuchsgruppe 2 | 30 | 8,25 | 27,03 | |

### Beispiel 7: Enzymatischer Abbau von Fumonisinen in der Bioethanolmaische

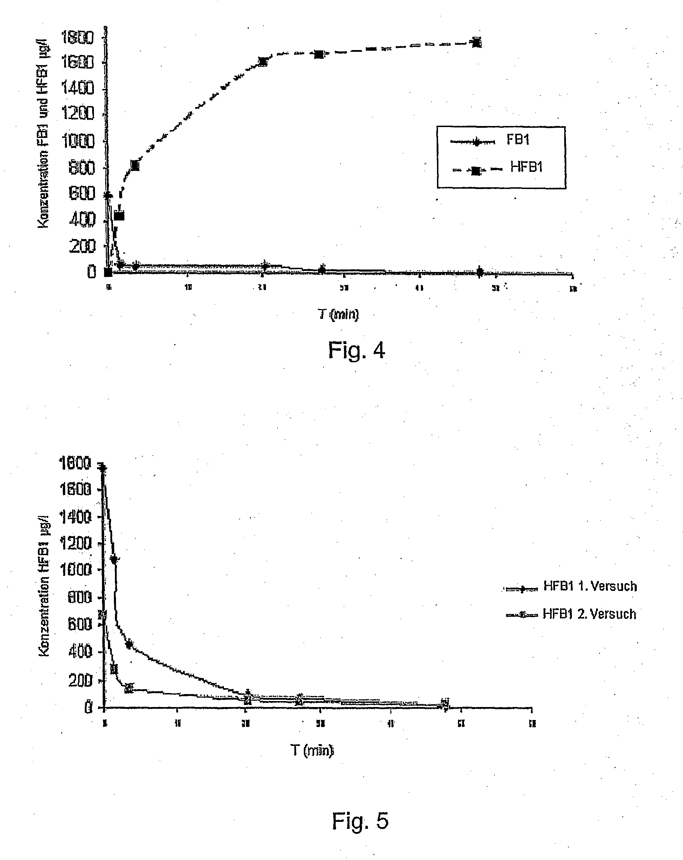

Proben von Maismaische zur Bioethanolherstellung wurden genommen und bei 30 bis 65 °C unter Rühren inkubiert, wobei der Abbau von Fumonisin B1 nach Zugabe von 770 Units Carboxylesterase ID-Nr. 9 pro Kubikmeter Maische unter Rühren (Rührzeit in Minuten) untersucht wurde. Proben wurden nach der Probennahme durch Aufkochen inaktiviert und danach für die Analytik abzentrifugiert und ein Aliquot des Überstandes abgedampft. Der Rückstand wurde in 200 µl Probenpuffer, der C13-markierten, internen Fumonisin-Standard enthielt, aufgenommen, 1,5 min geschüttelt, abzentrifugiert und dann einer LC-MS-Analyse unterzogen. Hieraus ergibt sich, dass, wie dies in Fig. 4 dargestellt ist, Fumonisin FB1 vollständig in hydrolysiertes Fumonisin HFB1 umgewandelt wird. Nach Zusatz von Aminotransferase ID-Nr. 19 wird das hydrolysierte Fumonisin HFB1 vollständig zu unschädlichen Bestandteilen abgebaut, wie dies in Fig. 5 dargestellt ist.

### Beispiel 8: Abbau von Fumonisinen und ihren Derivaten in Maistortillabrei und Cornflakesbrei

Die Wirksamkeit der Fumonisin-abbauenden Enzyme wurde in Maisbreiproben ("corn grits") für die Maistortilla- und Cornflakes-Herstellung untersucht. Fumonisin-kontaminierter Mais (ca. 1 ppm) wurde zu Maismehl vermahlen, mit Wasser versetzt und aufgekocht. Für die Tortilla**-**Herstellung wurde der auf ca. 50 bis 60 °C abgekühlte Maisbrei mit einer Mischung von Proteinasen in alkalischer Lösung versetzt. Nach 30 bis 180 min, wenn der pH unter 9, vorzugsweise unter pH 8, gefallen ist, wurde eine Mischung aus Carboxylesterase und Aminotransferase (jeweils 500 - 1000 Units/m³) hinzugefügt und für weitere 30 bis 60 min inkubiert. Im Falle der Cornflakes-Herstellung wurde ein Maisbrei aus gemahlenem Mais und Gerstenmalz für ca. eine Stunde im Druckgefäß aufgekocht; nach Abkühlung unter 60 °C (vorzugsweise 50 °C) wurde eine Enzymmischung, bestehend aus Carboxylesterase und Aminotransferase (jeweils 500 - 1000 Units/m³), hinzugefügt und für weitere 30 bis 60 min inkubiert. Aus dieser Mischung wurden dann Proben gezogen und auf FB1- bzw. HFB1-Rückstände wie in Beispiel 7 untersucht. HFB1-Levels waren in sämtlichen Proben unter 80 ppb, offensichtlich wurde das aus FB1 entstandene HFB1 kontinuierlich weiter umgesetzt. Die gemessenen Werte für FB1 sind in der u.a. Tabelle angeführt.

**Tabelle: Enzymatischer Abbau von FB1 und HFB1 in Maisbrei; Fumonisinkonzentration in ppb (µg/kg)**

| Behandlungszeit mit Enzymmix (min) | Tortillabrei (40 °C, 500 Units) | Tortillabrei (50 °C, 1000 Units) | Cornflakesbrei (35 °C, 500 Units) | Cornflakesbrei (40 °C, 1000 Units) |
|---|---|---|---|---|
| 0 | 852 | 866 | 912 | 1053 |
| 10 | 116 | 134 | 51 | 97 |
| 30 | 32 | 71 | 17 | 37 |

Organization Applicant
   Street : Industriestraße 21
   City : Herzogenburg
   State :
   Country : Austria
   PostalCode : 3130
   PhoneNumber :
   FaxNumber :
   EmailAddress :
   <110> OrganizationName : Biomin Holding GmbH
Application Project
   <120> Title verfahren zur Herstelllung eines zusatzstoffes für den enzymatischen Abbau von Mykotoxinen sowie Zusatzstoff und Verwendung desselben
   <130> AppFileReference : P04569
   <140> CurrentAppNumber :
      <141> CurrentFilingDate : __-_-_
Earlier Applications
   <150> PriorAppNumber : AT GM501/2008
      <151> PriorFilingDate : 2008-09-18
Sequence
   <213> OrganismName : sphingopyxis sp.
   <400> PreSequenceString :

tgtcggcgat crgtaaactt ctaccgtggt cctcgttcgc ccacakcata catcacagac 60
rtcgggattt ccaactgaac gggtcccggc ctgccggccc acatttcccg gaacgccata 120
tgggtgattt cgacaatccg gttccaggcg aagatgggtg cgccccattt aaccgcgggt 180
cgaaagaggt cgatctggtc ttgtccctga aaggtttttg gcgtgcaggg ataaacgaca 240
ccaagttgat gctgggacgt tattgcgacg aagggaaccc cttcgtggcg tgccgtcacg 300
actccaggca gaaggtttgc cgtaccggga cccggattcg tgacaatcgc ggcgacctgt 360
ccggtggtct tgtaaatgcc ctcggccata taggctgcgg cggcctcgtg ccgcaccggg 420
acgaacaata tcccattgtc ttcgagcgca gccaggagcg gatccacctc cggcgacatg 480
aggccgaaga cataccggac gccttcgacg gccaaacatc gtgccaataa ttctccgccc 540
gtgaggcgca tgacgatctc cagtacgaaa ggtgagtgcc caggttccgg cacattcgct 600
gtggttagtt gatgcgctga tcggccaacc gactgagtgg agttggatgg ccgcacctta 660
ccctgtcgcg cataactctc agatccggaa acggaccccg acattaaaat agcggccgac 720
cggatcatag gcagagctgg tcgggctgga aaaactgctg gggtcgttcg tcgctattgg 780
cggatctcgg tcgaacaaat tattgaccga tagaaacagc tgctgcttct ggccaaaagc 840
cgcgatgtcg aaggtcaatg tcgcgtcggt gtaccaaacc gccggagcgt ggttcaaatt 900
cgtatcgacg ccctccacat tgtcggcatt gaacaccgat gctgcgatga agcgctgctg 960
cacgagaagc gcccaatcgt cggtcgaata tcgcgcctgg aagttggccg accattttgg 1020
cgtgtccggt tgtccgagcg aacggatggg cgccgagccg gtcgcgatgc gataggcaga 1080
ggtatggtgc gttgccagcg cacgaagact gaacgtgccg ccgccgacgg ggcgtgagta 1140
ataggcctcg aagtcaattc ccgccgcttt ctggacagcc aggttgagat tgggacccgt 1200
cactgtgatg gtgccgtccg gattctccgt tatgaggtcg cagaagaagg tgtttcctgc 1260
atcgcacgcg tcgatttcct gctggggaag gaggaaatcg atcgcgccct tcaccttcac 1320
cacatagcga tcgaccgaaa actgaaaccc cggcacgaag gcggggcgta gcaccgcgcc 1380
gaatgtaagg acgtccgcct tttcagggcg caaatccgcg ttgccggcgg taaagaaccg 1440
cgtctgcaca gcctgtccgc cataaattga attgagcgtc gcctgacggc cggggtcgaa 1500
tagctcgaca aggcttggcc cgcggatatc tcgcgaacgg gtcgcgcgga acctgaggcc 1560
gtcgatcggc tcatattctc cgcccagctt ccaggttgtt actccaccgg actggctgta 1620
atcggcatat cggacggcgc cgtttaagtt cagcgaacgt cccagcgcgc tgtccttcag 1680
aatcgggacg ccgatttcga caaaaccttc cttgatgtca tagcttcccg agaaggaaag 1740
tgggttgtag agattgaagc ctccaggccg acctgcctgc gccgccggag ccccctgat 1800
tcccgtgatc gaggtcgtcg cctgcgatat cgcgtcggtt tcctgccggg ccttctcctt 1860
gcgatattcg ataccagcgg cgaccgagac cgggcccgcg ccgaacgaca ggctatcgcc 1920
gaggtcgccg gaaatcgtga gtcccgccac atattgctca agcctcagct gagcgacgcc 1980
atcagcggtg acatagtcga tggccgacgc gctcggcgag cctgtgccga agagattgag 2040
cggcacgcaa tcttggtcga ggccggccag tgttgaacgg cagacgatat tgcccgcggg 2100
atcgcggacc gcatcgacgg cggcgtagag attgcggttg atggtgagat tgttttcacg 2160
aagctcgagg tccgtaaggc caaaggaggc cgagccatcg agtttccagc cattgccaat 2220
gtctgcccgg aagccggcag cgccgcggta gacctttgcg aaattctcga tttcgaccaa 2280

gggaaagtcg cttgagaagc gaccgacaac gatcgaagcc tgggcatttc tgtccatgag 2340
cgtcgcgagt ggagccggaa ggaaggcgtt atcacggaag atccggaaat tattcgagcc 2400
accgacatgc gatattacga atgcacccag gttggtgtgg gaataagcat aggtgccctc 2460
cgcatacacc tgcacagtgt cggacacatc atatgcggcg cgtaggaacg cgttgtagcg 2520
aagctgatcc ggggcgaagc cgatattcac gcgcggtcca tcgccgccgc tctggaacga 2580
cgagctcgta aaattcccgt agtcgaaggt ccctaggact cctccgggca aaaacgcgat 2640
gcctttcaga gggccggacg tgacaagtcc gccgtaggat ccgcgagaac tgcgaatatc 2700
gggcacgacc gtgacgcctg tcgtagcgcc gggcacggga tattggccgg cggcgatgtc 2760
gaaccagcgg cgacccgttg cttcatcggc ccggattccg tcctgtcgaa aatattcgaa 2820
gctgccgagc aagtgcaacc ggtcgtcggc aaacgaagtg ccgaaggcga tcgaaccgcc 2880
gtaggacggg aggtcgccgc gggttgaaac acccgactgg agctcggccc tgatgccttc 2940
cagatcttcg tcgagcacga agttgatgac gcccgaaacg gcatcggaac cgtaggcggc 3000
cgaggcgccg cccgtcacga catcgacgcg cttgaccaac gcctgcggca gcacgttgat 3060
atcgaccgag cctgtgaaat tggtcgcgac gaaacggttg ccgttcagca ggacgaggtt 3120
ccggtttgac ccgaggccgc gcatgttgag caggttctga ccgctgttcc ccgttccggg 3180
tgtcgtgcca gggttggagg tcttcaagct gtcgttgaac acgggcagct ggttgagtgc 3240
gtcggcaagg ttggtcggag atgcctcctt caactgctcg ctggatacgg ctgtaaccgg 3300
cgtcggcgaa ttgaagccgt tctggaggcg gctgccggtc acgacgattt cgctcgttcc 3360
ccggtccgtg tccgcttcgt ccggctgacc tatcgatgcg ggatcgctat cctgagcact 3420
ggcagagaca ggaaatgcga gggtgccgag cgctactgcg ccgagcaaac tatttgcctt 3480
gccgggcttt tcgattctga acttccgata catctgcagt ccctcccgaa ttgataggga 3540
ctccgtttga gtccccttgt ttcttgacgc cgccgtcgct caccacggtc cggtcggagg 3600
ctaagcgtcg ggcctaagga cccgcaattt gaacatcaaa tgcaatgatc ggaggcttca 3660
ttgcacttcg cgcatagacc ggcgcggtag ctgaaagtgc caataatcag ggattttgct 3720
gaacagttgc ggcatgacgt ccggcatcgg ccacgcggtt ggcggcatcg acgtggcttt 3780
cgcgtcgccg cccctcaagc accggcgagt tgcattaaaa tgggatgagg ctggagagac 3840
gcaaaatctc tgaggaccgc gctgaacgcg cgatccgtcg cctcgagggt ctccgttaca 3900
tcgtcaactg tatgggccgc agagagaaac atattgtgat agggatgaac atagacgccg 3960
cccttcaggc acgccgcggc ccacgcatag ccgatccgaa aatcgggatc gtccgcaaag 4020
aatatttgcg gcatctgcgc cgggcccgtc tgcttcaact caagaccatg gcgctgagac 4080
tgtgcctcca ggcctgcccg cagggcggcg ccgctggcga tcagcgtttc gagataaggc 4140
gtctctcgaa tgatcctgag ggtttcgatc gcggccgcca tcggtaccgc agagaaccag 4200
aaggagccgg tcacaaatat atcccgcgcc gcatcgcgcg ccttgttcga gcccagcagg 4260
gcggagatcg gatagccatt cgcaaagcat tttccccagc aactgagatc gggttcgata 4320
cccaaatgcg tccagctgca atcgcgcgcc acccggaaac ctgcgcgcac atcgtcaacg 4380
accagaagcg caccggtctc gtcacaacat tttcgagcgg tgcgcgcgaa ctcaagctgg 4440
gcgagggcct ggtcctcaaa tacttcgtgt cggaaaggtg tggcaaagac agccgcaata 4500
tcgccatcgt gcgccttgaa cgcgtccgat aagctttggg cgtcgttata ggtataatat 4560
gcgacatgca cgcgatcgga agcgagaatc ccggcagtat gcggagtgtt ccacggggaa 4620
gcgccatgat aggcgccttt ggcgcataat atggttttgc gccccgtatg ggcacgcgcg 4680
agaaccatcg ccgttgaggt ggcatcgctg ccatttttgc agaacatcgc ccaatccgca 4740
tgacggacca tgcccacaaa ggcttcggcg aggttgacca tgatctccga aggaccggtc 4800
atggtgtcgc cgagaagtcg ctgcgcatca gccgcggctt cgatttcgga ttgccggtaa 4860
ccgagcaaat ttggcccata cgcgcacata tagtcgatat agggctgctc gtcggcgtcc 4920
caaattcgtg cccccagcgc gcgcctgaag aactggggga attctggcgg cagcaaccgt 4980
gtcgactcgt ggccgtacat cccgcccgga atgacccgtt cggcgcgttc tctgagatct 5040
ttctgccttg ttccgttcgc cataatgcac ctctcgcgat aaataatggg taaaaatcca 5100
cgaaattcaa cgattcgtga tctgaaagag atatatcttg taatatactg tataattata 5160
cacaatgcgc aatcggacga cgggatagcg gggcagggag gacggggaaa tctatgcgga 5220
acgtcagcga caaggcgccg ccccacgaga cgctcaccgt agtcgtcgcg gcaatgatcg 5280
ttggcacggc cgccttgatg gtgcttggaa tacagcccat ccttctcggc gcccttgtag 5340
aggaggggcg tattcccgcc gaggggttgg gatcggcggc aacggtggaa atactggcga 5400
tcgcggcggg aacatgcatc ggacccgttc ttatgaagac gggatatctg cgggcgaaat 5460
gcgcggcact ctgcttaatg ctcgccgcaa tcaacttcgg attgacgttg ccgggtttcg 5520
atttgcccat cgtggcttgc cgagcggcag cgggagccct ggaaggtctt tcgctcagcg 5580
cggcgatcct gatcatgact cataatcggc ggccggaccg gctgagcgga atatttctgg 5640
gcgcgcagac gataccgcag gtaatatctg cttatttgct cccgacggag attattccgc 5700
gctgggggag cgcaggcggc ttcacgatcc tgggcattct cgcggcgatc gccgcgatcg 5760
cggctctgtg cctcgtcgat cgcgttgagc tcgatccgac gaccgttaac gacgacttgc 5820
agtggtcacc cgcggcgatc gtcatttcga tggcggcatt cgttcaattc tcgggggtcg 5880
gtgccgcatg gagctatctg gagcgactgg ctgcgcagca cggattttcg ggagaaacga 5940
tcggtatcgc catttccggg agtttgcttt gccaggtagg cggggcttgg ctggccgctt 6000
ggatcggtgg gcgggtcgga tatcgcttcg ccttaatcgc tgggagcctg cttcaggcgg 6060
gcaacgtgat cgcattggcg gtggccgatc agccaagctg gtttatttcc gcttcctgtg 6120
ctttcggcct gttctggttg gcgatgcagc ccttccaaat ccgcttcgcg atcgcgatag 6180
ataacagccg gcagcttgct gtactgctga cgccgatcgc cctcgtcggg ttgagcgcgg 6240
ggcccttgtt gctctctcgc tttgccgggg cgaccgactt gcgctggatc tttgtgggga 6300
gttcgacctt gttgctggcc agcgcgcttc tgtatctttg cgcttctctg tttcaaccgc 6360

gcggaaaggt gatcgctgaa acggtggacg tatgaaaaag acggatcggg gttcgcgatg 6420
acatcgcagg tcaagcttcg tagcgcggca aagcggccgc gcagtcctaa aagcgagcga 6480
ggtcttgctc gttacgagtc cttgcttgat gcgaccgaca ggctgttggt cgatctagac 6540
cccgatcagg tcggtctcta tcagattgca gaggaagcgg gtgcctcacc gtcgtccgtc 6600
tatcatttct ttccgaccaa ggaagtggct catctcgctc tgatgcgccg ctatctggag 6660
gggctccgga atctcgacgc gatggaagtc gacatcggcc agctcgaaag ctggcaggac 6720
ctgatgaagt tggatcagat cagggcgcga gactattata atagccaccc gcccgccctc 6780
aagcttctgt tcggcggata tggcggggtc gaggccagaa agcttgacga gcgatactcc 6840
gaggaaatcg tgagctccat gtatggcaga tacaacggca ttttccatat gccgcaaatg 6900
gagaatgagg ctctcatgtt cacgatctgc ttcgcaattc tcgacgcggt atgggccgtc 6960
tcctttcgcc ggttcggtga aattacgtcg gattttcttc gggaggggca agcggcttgc 7020
attgcctatt gccgacacta tctgcccgag cgaacgccat cagcgtgaat ccgttcaacg 7080
atatgcagga atgtccgttg cgttgagttc ggttctgagt tcggtcggtt aggaggcccc 7140
gcgataaacc aacgctcttc tgtcgaaggg atgtcgcctg gttcgaccag gccctgcgaa 7200
gtcagccgca atcaacgagg cagatgtcaa cgtggccagc aagttcaact gtgagttact 7260
cgatctgcga tcatttgttg cggtgtatga aacgcgaagt tttagccacg ccgcgcggct 7320
tctgaatcaa tcgcagcccg cgctcagccg gagaatccag cgcctcgaga gtctcgtggg 7380
cggtccgttg ttcgagcgga ccagtcggtc gcttgccgaa acggcgctcg gcaaagagtt 7440
gctcccggtc gcccaccgag cgttggaact tgtcgatacg tcgctgtttg cgtcgcccaa 7500
tgtccgggag ttccgctgga cagacatcac gattgcctgt gtacagaccg ccgccttcca 7560
tgttctcccg cgagctgcgc gcttgtacat ggatcaaaat ccgagggtcc gactccgcat 7620
ccttgacgtg ccggcggtcg aggctgcgga cctggttgcg agcggcgagg cggagttcgg 7680
catcagcatt gagagcctgt tgccatcaag cctgcggttc gatgcgctcc acgaggaccc 7740
gttcggcctg gcatgccacc gaagccatcc gctggcgtcg ctcgagatcc ttgaatggac 7800
gcaattgaaa ggtgaaagcc tgatcgccgt tcaccgtgcg agccggaacc gcacgttgct 7860
cgatgccgaa ctcgcgcgca acaatatcgc gctggaatgg cggtatgagg tcgcgcatct 7920
gacgacggcg ctgggattga tcgatgcgca attgggtgtc gctgttatgc cccgcatggt 7980
tatgccccgc tcgggtcggt cggaggtcgt ctggcgcccc gtcgtcgcgc cggtcgtcca 8040
acgcacgatc ggcatcgttc agcgccgcac cggctcgatg caccctgccg cacagcaatt 8100
gcttgcgcgg ctccgcgcgg cctggtcgtc cgccaatctg ggcgacatcg cgtctcgcga 8160
agatggggca tcgtgacacg cgttctatgc gcctgcagca tcgatgctca cgatcattgc 8220
atttgctgag agacgaacgc gaagataccg ctgggtcaca ggatatcagt ccatcgaggc 8280
gggagagaaa tgtgtgaaag agcaccaatg ccgtggcggc cgggcgtccc ccgctgcgcc 8340
cgccacgtgg cttgcgcgga tcagcgtttc ccggggggcc tccgccatcg cctggacctt 8400
catgcttggc gcaactgcca ttcccgtggc tgcgcaaact gacgatccga agctcgttcg 8460
tcatacccag tcgggcgccg tcgagggcgt cgagggcgac gtcgagactt ttttgggaat 8520
acccttcgcg gctccgccgg tcggcgacct gcgatggcgg ccgccggctc cgccgagggc 8580
gtgggcgggc accaggacg gccgccgctt tgcgcccgat tgcatcggga acgagcggct 8640
tagagagggg agccgggctg ccgggacgag cgaagactgc ctctatctga atatctggtc 8700
tcccaaacag gtcggtaagg ggggctccc cgtcatgatc tgggtttacg gcggtgggtt 8760
tagcggcggt tctggcgcgg tgccatatta tgacggctct gcgctcgcgc agaagggcgt 8820
ggtggtcgtc acgttcaact atcgcgccgg gattctgggc tttcttgccc atccggcgct 8880
ttcaaaggaa agtccgaatg gcgtgtcggg caactatggt cttctcgaca tgctcgcggc 8940
gttcaaatgg gttcagaaca acataaggga gttcggcgga gacccgaacc gtgtcacggt 9000
ctttggcgag tccgccggcg cgagcgcgct cggactgctc ctgacctcgc cgctcagtga 9060
gagcgccttc aatcaggcga tactgcaaag tccgggtctg gccaggccgc tcgccacgct 9120
ttctgaaagc gaagcgaatg ggctggagct gggagccgat atttctgctc tacggcgtgc 9180
cgatgcgggc gaattgacga agatcgcgca atcgcgaata cccatgtcgc gccagttcac 9240
caagccgcgg ccgatgggtc cgattctgga cggctatgtt ttgcgcaccc ttgacgtcga 9300
tgccttcgcc aagggggcct tccgcaagat acccgttctg gtcggcggaa acgccgacga 9360
agggcgcgct tttacggatc gcctgccggt caaaacggtc cttgaatatc gagcctatct 9420
cacagaacaa tttggtgacg aggcggacgc atgggagcgt tgttatcccg cgaactccga 9480
cgccgacgtc cccgccgccg ttgcccgtct tttgggggat agtcagttca acaacgggat 9540
cgagctgctc tcggcagcct tcgcgaaatg gcgaacgccg ctttggagat atcgctttac 9600
gggcattcca ggagccggcc gtcgccccgc cacgcatgga gacgaaattc cctatgtctt 9660
cgcaaatctg ggccgtcgt ccgtatctat gtttgggtcg ctcgaaggcg gcgccgggc 9720
gtcggacatc aaacttgcga ccgaaatgtc cgcggcctgg gtgagcttcg cggtgcacgg 9780
ggtccccgat cagggcacga aatcgcactg gccgcgcttc gagcggcgag gggagatcat 9840
gacttttggt tcgcaggttg gctctgggga aggtcttgga gtttcgccga gcaaagcctg 9900
ccaaccctca aaatagcgcc cggcctgtgc gtgcttcagc acgccgtccc gctttgcggg 9960
cgacgggctg tgccctctgc ctagaaggaa gtaagttgcg ctacgacgtc gcgataattg 10020
gaggtggcaa cgctgcattg acggcagccg tgacggcgcg tgaagcgggg gcctcggttc 10080
ttgtgatcga gcatgcgccg cgcgccatgc gcggcggcaa cagtcgtcac acacgcaata 10140
tgcgtacgat gcacgaacgt cccctgtcgc cgttgaccgg tgaatattcg gcggacgaat 10200
attggaatga tcttgtccgc gtcacggggg ggcgcaccga cgaagaactc gcgcggctcg 10260
ttatccgcaa caccaccgac gctattccct tcatgacgcg ctgcggtgtg cgtttccagc 10320
cctcgctgtc gggcacgctg agtttatcgc gaaccaacgc attcttcctt ggcggcggga 10380
aggcgcttgt aaacgcatat tacgccacgg ccgaacggct aggcgtcgat attctctatg 10440

attctgaggt gaccgagatc aaccttcagc aaggcgtcgt gcagcgtctg caattgcgca 10500
gccggggatt ccctgtcgaa gtggaagcca aggctgccat cgcctcgtcc ggaggattcc 10560
aggcaaatct tgactggctc tcaagcgcat gggggcctgc tgcggcgaac ttcatcgtac 10620
ggggcacgcc atatgcgact ggcacggtgc tcaagaacct gttggagcaa ggcgtcgcct 10680
cggtgggaga tccaacccaa tgccatgctg tcgcgatcga tgggcgagcg cccaaatacg 10740
acggcggcat cgtcacacga ctggactgcg ttcccttctc gatcgtcgtc aacaaggacg 10800
ccttgcgctt ctacgatgaa ggcgaagatg tgtggccgaa gcgttacgcc atatggggtc 10860
gcttggtggc acagcagcct gatcagatcg ctttcagcat aatcgatcgg caggccgaag 10920
acctcttcat gccgtcagtg ttcccccccg tgcaagcgga cacgatcgcg ggtctggccg 10980
agaaactcgg tctgaatccc gtaaccctgg aacgcacggt ggccgaattc aacgccgcat 11040
gcgtgcccgg cgaattcggc ggccaagatc tcgacgacct ccacaccgag ggaatcgaac 11100
caaagaaatc caactggccc cgaccgatta ttgtgccccc gttcagcgcc tatcctctcc 11160
ggcccgggat caccttcacc tatctcggcg tcaaggtaga cagccgtgcg cgggtcatca 11220
tggagacagg tgagccgaca aaaaacctgt ttgcttcggg ggaaataatg gcgggcagca 11280
ttctcggcca aggttatctc gctggatttg gaatggcgat tggtaccgta ttcggccgca 11340
tcgcgggttg ggaggccgca cgtcatgcag gattttgatc tcgtaaaaat gctgtctgac 11400
ttgccgtcgg cgccggagct ggaagccagg cgcgttatgg aggtgtgcaa cgcgtgccgc 11460
tattgcgaag ggttctgcgc ggtatttcct gcaatgacct tgcagcgtca tttcgccagc 11520
ggcgatctca gccacctcgc caatctctgc cactcgtgcc aaggttgcta ttacgcctgc 11580
caatacgccc ctccgcatga gttcggaata aacgttccaa aggcgctgtc ggagttgcgg 11640
ctcgagagct acgagcagca tgcttggccc cggccggtcg ccgctctcta tcgcaagaat 11700
gcgctcatca tttccatctt gtcggcggca tgcataaccg gcgtccttct gcttgccgcc 11760
atcttcaacg ggatgcact tttcgcgaaa cacgcatcgg tgcccggcgg cgggttttac 11820
aacgttattc cttatcaggc gatgattgcc gtcgcggcga ccacatttct ttattccgcg 11880
ctggcgctgg cgatcagtct cgttcgcttt tcgcggacga tcggtctggg aattaaggtt 11940
ctttatcagc acgtgccggt tcttcgggcg ctacgcgatg cggcgactct gcgatatctc 12000
ggcggcagcg acggcgaggg gtgtaacgac gcggacgaga cattttcgac gacccggcga 12060
aaatttcatc acgcccttgc ctatggcttc ggactttgtt tcgcggccac agccacgggc 12120
acgatctacg atcatatgtt cggctggccg gcgccctatg cgcttttcag cttgccggtc 12180
gtcctaggga ccgttggggg gatcggaatg gtcgtgggcg cgatcggcct actctggctc 12240
aagctggccg gcgaagacgc tcctcgatca ccggcactgc ttgggccgga tgttgccctg 12300
ttggtgcttc tgcttgccat agcggcaacg ggcctcctcc ttttagcggt ccgcagcacc 12360
gaagtcatgg gcgtcgcgct cgccgtccat ctcggcgtcg tcttggcctt ctttttggtg 12420
atgccataca gcaaatttgt ccacggtatc ttcaggctca cggctctcgt gcgccatcat 12480
gctgaccgcg aggcaagtaa tggcttcgcc tccagccctc ccacgaaaaa gggttaaaca 12540
atggaacata tgaagtccgt tcgcgatcgc agtagcgtca tgcagatcgt gagagtggcg 12600
agtggcaact gtctcgagca atatgatttc ttcgtttacg gcttctatgc ggcatatatt 12660
gcgagaagct tttttccgac cggcgataac gcgacatcgc tcatgctttc attggccact 12720
tttggcgctg gtttcctcat gaggcccttg gggcgatttt ttctcgggtc ctacatcgat 12780
cgcgtcgggc gtcggaaagg cctgatcgtg acactcgcga tcatggccgt cggaaccctc 12840
accattgcga tgactccaag ctatgaggca attggattac tcgcaccggt tatcgtgctc 12900
gtcgggcgac ttttgcaggg tttttccgct ggagcagagt cgggtggcgt ctcagtgtac 12960
ttggcggaaa ttgcgtcgcc caaatcgaga ggcttcttca cctcgtggca gtctgccagc 13020
cagcaggtgg ccgtcatgat cgccgccgcg atcggtcttg cgctgcaatc aacgctttca 13080
ccggagcaaa tgaacgactg ggatggcgg gtgcccttgt tgatcggatg cttgattatc 13140
cccgtgatac tctggctgcg ccggtctctc ccggaaacga aagcctatct ccacatggag 13200
cacaaggcgc attcgatcgg cgaatccctc cgcgaattgc aacagagctg gggctgatc 13260
ttgacgggca tggcgatgtc gatcctcacg acgaccacct tttacatgat taccgcctat 13320
acgccgacat ttggcgagaa agcactcgga ctgagcccgc aagatgtcct gctggttacc 13380
atcatggtcg gcgtgtcgaa cttcctgtgg cttccgatcg gggtgctct ctcggatcgt 13440
atcggtagaa ccccgatcct actggtcgtg ccggtcaccg ttctcgccat cgcctttccc 13500
ctgatgagct ggctcgtcgc ggcaccgaca ttcggagcgc ttgcagctgt tctgctgact 13560
ttctccgcat gctttggact ctataatggg gcgctcatcg cgagactcac cgagattatg 13620
cctcccgcca ttagaaccct tggcttctcg ctggcgttca gtctcgcgac ctcgctgttc 13680
ggcggcttca ccccattggt aagtacggcg ctaatccacg cgacgggcag caattccgcg 13740
cctgcaatct ggctctgttt tgcggctttc atcagcttcg tcggtgtggc cgcatcgacc 13800
cggctgagcc ggccaatcgc cgaaggcgcc agataggaca atcagagaat gcccgtgcgg 13860
caatgaagcg agattcgggc ggtaggtgcg ctggcggcac ttcgcgaaga gccgttgcgg 13920
acggctgaaa cgatgatggt atgaatgggc taagacatga gagcagtagt ttaccgaaat 13980
ggcgaacttg tcctgggggc ctatgctgat ccgatacccg ccgccgggca ggtgctcgtc 14040
aagaccagag catgcggcat ctgcggatct gaccttcatt tttgcgatca tgcgcaggcg 14100
tttacgaacc ttgcatcgcg ggcgggtatc gcctctatgg aagttgattt gtgtcgagac 14160
atcgttctgg ggcatgaatt ctgtggcgag attatggagt tcgggccctc tgcggatcgt 14220
cgcttcaaac ccggacagct tgtgtgctcg ctgccgctgg cgatcggtcc gaccggagcg 14280
cggacgattg gctactcgga tgagtatccc ggcgggctcg gcgaatatat ggtcctcacg 14340
gaagcgctct tgctgcctgt tccgaacggc cttccggcga cctgcgcggc gttgacggag 14400
ccgatggcgg tgggatggca tgccgtcgag atcgcgcagg ttcaaccaca tcacatccct 14460
gtggtgatcg ggtgcggacc ggtcgggttg gcagtcgtcg ctgccctgaa acataagcaa 14520

gttgctccga ttattgcgtc ggatccatcg cccgatcggc gtgctcttgc tctgcggatg 14580
ggcgccgacg ccgttgtcga tccgcgcgaa gaatcaccct ttcgccaggc cgagaagatc 14640
gcacgcccgg tcggacaagg tggggccctg tccagctcat tgctgtcaaa gtctcaaatg 14700
atattcgaat gcgtaggggt gccgggcatg cttcggcatg cgatggacgg cgcgtccgac 14760
gggtccgaga tcatggtcgt tggcgcatgc atgcagccgg acgcgatcga gcccatgatc 14820
gggatgttta aagcgctcac gatcaaattc tcgcgaactt acacgggtga ggaattcgcc 14880
gcggtgcttc acatgatagg tgagggcgca ctcgacgtat ctccgctcgt taccgatgtg 14940
attggcctgt ccgatgtccc gtccgcgttt gaggctctac ggagtccagg cgcccaagca 15000
aaagtgattg tggacccttg gcgctgagcc tgaggatgcc aagggtgcga cgttgggcat 15060
cgtcaaagaa ggcgacgttg acccggtatg tgaacatccc catattcttc cgcagctgaa 15120
gcagttggta aacatgccaa aatatgaact gtagtattgc gtcggggttc tcattgtggg 15180
gtttgccatt gtcatcgctc gcacccggcg acaaagatta gatgtacttc cgataatccg 15240
tgctctcgac ctggccttcc ttcatatatt tcaggacctc tccgaccatg cgtgcggcgc 15300
ggatcgggat cggcaggcgt tggttcatct gggtcgagtt ccagttgatc ttcgtaagag 15360
agaacacctc ctcggctaac tgcgccgcgg tactatcgca ggatcgtctc gagcgtycgc 15420

   <212> Type : DNA
   <211> Length : 15420
      SequenceName : sequence 1
      SequenceDescription :
Feature
sequence: sequence 1:
<221> FeatureKey : Seq ID 1 (fum)
   <222> LocationFrom : 1
   <222> LocationTo : 15420
   Other Information :
   CDSJoin : No
Sequence
<213> OrganismName : Sphingopyxis sp.
<400> PreSequenceString : 
atgcggaacg tcagcgacaa ggcgccgccc cacgagacgc tcaccgtagt cgtcgcggca 60
atgatcgttg gcacggccgc cttgatggtg cttggaatac agcccatcct tctcggcgcc 120
cttgtagagg aggggcgtat tcccgccgag gggttgggat cggcggcaac ggtggaaata 180
ctggcgatcg cggcgggaac atgcatcgga cccgttctta tgaagacggg atatctgcgg 240
gcgaaatgcg cggcactctg cttaatgctc gccgcaatca acttcggatt gacgttgccg 300
ggtttcgatt tgcccatcgt ggcttgccga gcggcagcgg gagccctgga aggtctttcg 360
ctcagcgcgg cgatcctgat catgactcat aatcggcggc cggaccggct gagcggaata 420
tttctgggcg cgcagacgat accgcaggta atatctgctt atttgctccc gacggagatt 480
attccgcgct gggggagcgc aggcggcttc acgatcctgg gcattctcgc ggcgatcgcc 540
gcgatcgcgg ctctgtgcct cgtcgatcgc gttgagctcg atccgacgac cgttaacgac 600
gacttgcagt ggtcacccgc ggcgatcgtc atttcgatgg cggcattcgt tcaattctcg 660
gggtcggtg ccgcatggag ctatctggag cgactggctg cgcagcacgg attttcggga 720
gaaacgatcg gtatcgccat ttccgggagt ttgctttgcc aggtaggcgg ggcttggctg 780
gccgcttgga tcggtgggcg ggtcggatat cgcttcgcct taatcgctgg gagcctgctt 840
caggcgggca acgtgatcgc attggcggtg gccgatcagc caagctggtt tatttccgct 900
tcctgtgctt tcggcctgtt ctggttggcg atgcagccct tccaaatccg cttcgcgatc 960
gcgatagata acagccggca gcttgctgta ctgctgacgc cgatcgccct cgtcgggttg 1020
agcgcggggc ccttgttgct ctctcgcttt gccggggcga ccgacttgcg ctggatcttt 1080
gtggggagtt cgaccttgtt gctggccagc gcgcttctgt atctttgcgc ttctctgttt 1140
caaccgcgcg gaaaggtgat cgctgaaacg gtggacgtat ga 1182
<212> Type : DNA
<211> Length : 1182
   SequenceName : sequence 2
   SequenceDescription :
Feature
sequence: sequence 2:
<221> FeatureKey : Seq ID 2 (fumA)
   <222> LocationFrom : 1
   <222> LocationTo : 1182
   Other Information :
   CDSJoin : No
Datebase
Sequence: sequence 2:
<308> DBAccessionNumber : FJ426269
   <309> DBEntryDate : 2009-06-11
   <313> From : 1
   <313> To : 1182
Sequence
<213> OrganismName : Sphingopyxis sp.
<400> PresequenceString : 
MRNVSDKAPP HETLTVVVAA MIVGTAALMV LGIQPILLGA LVEEGRIPAE GLGSAATVEI 60
LAIAAGTCIG PVLMKTGYLR AKCAALCLML AAINFGLTLP GFDLPIVACR AAAGALEGLS 120
LSAAILIMTH NRRPDRLSGI FLGAQTIPQV ISAYLLPTEI IPRWGSAGGF TILGILAAIA 180
AIAALCLVDR VELDPTTVND DLQWSPAAIV ISMAAFVQFS GVGAAWSYLE RLAAQHGFSG 240
ETIGIAISGS LLCQVGGAWL AAWIGGRVGY RFALIAGSLL QAGNVIALAV ADQPSWFISA 300
SCAFGLFWLA MQPFQIRFAI AIDNSRQLAV LLTPIALVGL SAGPLLLSRF AGATDLRWIF 360
VGSSTLLLAS ALLYLCASLF QPRGKVIAET VDV 393
<212> Type : PRT
   <211> Length : 393
   SequenceName : sequence 3
   SequenceDescription :
Feature
Sequence: sequence 3:
<221> FeatureKey : Seq ID 3 (FumA)
   <222> LocationFrom : 1
   <222> LocationTo : 393
   Other Information :
   CDSJoin : No
Datebase
Sequence: sequence 3:
<308> DBAccessionNumber : FJ426269
   <309> DBEntryDate : 2009-06-11

   <313> From : 1
   <313> To : 393
Sequence
<213> OrganismName : Sphingopyxis sp.
<400> PreSequenceString : 
atgacatcgc aggtcaagct tcgtagcgcg gcaaagcggc cgcgcagtcc taaaagcgag 60
cgaggtcttg ctcgttacga gtccttgctt gatgcgaccg acaggctgtt ggtcgatcta 120
gaccccgatc aggtcggtct ctatcagatt gcagaggaag cgggtgcctc accgtcgtcc 180
gtctatcatt tctttccgac caaggaagtg gctcatctcg ctctgatgcg ccgctatctg 240
gaggggctcc ggaatctcga cgcgatggaa gtcgacatcg gccagctcga aagctggcag 300
gacctgatga agttggatca gatcagggcg cgagactatt ataatagcca cccgcccgcc 360
ctcaagcttc tgttcggcgg atatggcggg gtcgaggcca gaaagcttga cgagcgatac 420
tccgaggaaa tcgtgagctc catgtatggc agatacaacg gcattttcca tatgccgcaa 480
atggagaatg aggctctcat gttcacgatc tgcttcgcaa ttctcgacgc ggtatgggcc 540
gtctcctttc gccggttcgg tgaaattacg tcggattttc ttcgggaggg gcaagcggct 600
tgcattgcct attgccgaca ctatctgccc gagcgaacgc catcagcgtg a 651
<212> Type : DNA
   <211> Length : 651
   SequenceName : sequence 4
   SequenceDescription :
Feature
Sequence: sequence 4:
<221> FeatureKey : seq ID 4 (fumB)
   <222> LocationFrom : 1
   <222> LocationTo : 651
   Other Information :
   CDSJoin : No
Datebase
Sequence: sequence 4:
<308> DBAccessionNumber : FJ426269
   <309> DBEntryDate : 2009-06-11
   <313> From : 1
   <313> To : 651
Sequence
<213> OrganismName : sphingopyxis sp.
<400> PreSequenceString : 
MTSQVKLRSA AKRPRSPKSE RGLARYESLL DATDRLLVDL DPDQVGLYQI AEEAGASPSS 60
VYHFFPTKEV AHLALMRRYL EGLRNLDAME VDIGQLESWQ DLMKLDQIRA RDYYNSHPPA 120
LKLLFGGYGG VEARKLDERY SEEIVSSMYG RYNGIFHMPQ MENEALMFTI CFAILDAVWA 180
VSFRRFGEIT SDFLREGQAA CIAYCRHYLP ERTPSA 216
<212> Type : PRT
   <211> Length : 216
   SequenceName : sequence 5
   SequenceDescription :
Feature
Sequence: sequence 5:
<221> FeatureKey : Seq ID 5 (FumB)
   <222> LocationFrom : 1
   <222> LocationTo : 216
   Other Information :
   CDSJoin : No
Datebase
Sequence: sequence 5:
<308> DBAccessionNumber : FJ426269
   <309> DBEntryDate : 2009-06-11
   <313> From : 1
   <313> To : 216
Sequence
<213> OrganismName : sphingopyxis sp.
<400> PreSequenceString : 
gtggccagca agttcaactg tgagttactc gatctgcgat catttgttgc ggtgtatgaa 60
acgcgaagtt ttagccacgc cgcgcggctt ctgaatcaat cgcagcccgc gctcagccgg 120
agaatccagc gcctcgagag tctcgtgggc ggtccgttgt tcgagcggac cagtcggtcg 180
cttgccgaaa cggcgctcgg caaagagttg ctcccggtcg cccaccgagc gttggaactt 240
gtcgatacgt cgctgtttgc gtcgcccaat gtccgggagt tccgctggac agacatcacg 300
attgcctgtg tacagaccgc cgccttccat gttctcccgc gagctgcgcg cttgtacatg 360
gatcaaaatc cgagggtccg actccgcatc cttgacgtgc cggcggtcga ggctgcggac 420
ctggttgcga gcggcgaggc ggagttcggc atcagcattg agagcctgtt gccatcaagc 480
ctgcggttcg atgcgctcca cgaggacccg ttcggcctgg catgccaccg aagccatccg 540
ctggcgtcgc tcgagatcct tgaatggacg caattgaaag gtgaaagcct gatcgccgtt 600
caccgtgcga gccggaaccg cacgttgctc gatgccgaac tcgcgcgcaa caatatcgcg 660
ctggaatggc ggtatgaggt cgcgcatctg acgacggcgc tgggattgat cgatgcgcaa 720
ttgggtgtcg ctgttatgcc ccgcatggtt atgccccgct cgggtcggtc ggaggtcgtc 780
tggcgccccg tcgtcgcgcc ggtcgtccaa cgcacgatcg gcatcgttca gcgccgcacc 840
ggctcgatgc accctgccgc acagcaattg cttgcgcggc tccgcgcggc ctggtcgtcc 900
gccaatctgg gcgacatcgc gtctcgcgaa gatggggcat cgtga 945
<212> Type : DNA
   <211> Length : 945
   SequenceName : sequence 6
   SequenceDescription :
Feature
Sequence: sequence 6:
   <221> FeatureKey : Seq ID 6 (fumC)
   <222> LocationFrom : 1
   <222> LocationTo : 945
   Other Information :
   CDSJoin : No
Datebase
Sequence: sequence 6:
   <308> DBAccessionNumber : FJ426269
   <309> DBEntryDate : 2009-06-11
   <313> From : 1
   <313> To : 945
Sequence
<213> OrganismName : Sphingopyxis sp.
<400> PreSequenceString : 
VASKFNCELL DLRSFVAVYE TRSFSHAARL LNQSQPALSR RIQRLESLVG GPLFERTSRS 60
LAETALGKEL LPVAHRALEL VDTSLFASPN VREFRWTDIT IACVQTAAFH VLPRAARLYM 120
DQNPRVRLRI LDVPAVEAAD LVASGEAEFG ISIESLLPSS LRFDALHEDP FGLACHRSHP 180
LASLEILEWT QLKGESLIAV HRASRNRTLL DAELARNNIA LEWRYEVAHL TTALGLIDAQ 240
LGVAVMPRMV MPRSGRSEVV WRPVVAPVVQ RTIGIVQRRT GSMHPAAQQL LARLRAAWSS 300
ANLGDIASRE DGAS 314
<212> Type : PRT
   <211> Length : 314
   SequenceName : sequence 7
   SequenceDescription :
Feature
Sequence: sequence 7:
<221> FeatureKey : Seq ID 7 (FumC)
   <222> LocationFrom : 1
   <222> LocationTo : 314
   Other Information :
   CDSJoin : No
Datebase
sequence: sequence 7:
<308> DBAccessionNumber : FJ426269
   <309> DBEntryDate : 2009-06-11
   <313> From : 1
   <313> To : 314
Sequence
<213> OrganismName : Sphingopyxis sp.
<400> PresSequenceString : 
gtgaaagagc accaatgccg tggcggccgg gcgtcccccg ctgcgcccgc cacgtggctt 60
gcgcggatca gcgtttcccg gggggcctcc gccatcgcct ggaccttcat gcttggcgca 120
actgccattc ccgtggctgc gcaaactgac gatccgaagc tcgttcgtca tacccagtcg 180
ggcgccgtcg agggcgtcga gggcgacgtc gagacttttt tgggaatacc cttcgcggct 240
ccgccggtcg gcgacctgcg atggcggccg ccggctccgc cgagggcgtg ggcgggcacc 300
agggacggcc gccgctttgc gcccgattgc atcgggaacg agcggcttag agaggggagc 360
cgggctgccg ggacgagcga agactgcctc tatctgaata tctggtctcc caaacaggtc 420
ggtaaggggg ggctccccgt catgatctgg gtttacggcg gtgggtttag cggcggttct 480
ggcgcggtgc catattatga cggctctgcg ctcgcgcaga agggcgtggt ggtcgtcacg 540
ttcaactatc gcgccgggat tctgggcttt cttgcccatc cggcgctttc aaaggaaagt 600
ccgaatggcg tgtcgggcaa ctatggtctt ctcgacatgc tcgcggcgtt caaatgggtt 660
cagaacaaca taagggagtt cggcggagac ccgaaccgtg tcacggtctt tggcgagtcc 720
gccggcgcga gcgcgctcgg actgctcctg acctcgccgc tcagtgagag cgccttcaat 780
caggcgatac tgcaaagtcc gggtctggcc aggccgctcg ccacgctttc tgaaagcgaa 840
gcgaatgggc tggagctggg agccgatatt tctgctctac ggcgtgccga tgcgggcgaa 900
ttgacgaaga tcgcgcaatc gcgaataccc atgtcgcgcc agttcaccaa gccgcggccg 960
atgggtccga ttctggacgg ctatgttttg cgcacccttg acgtcgatgc cttcgccaag 1020
 
ggggccttcc gcaagatacc cgttctggtc ggcggaaacg ccgacgaagg gcgcgctttt 1080
acggatcgcc tgccggtcaa aacggtcctt gaatatcgag cctatctcac agaacaattt 1140
ggtgacgagg cggacgcatg ggagcgttgt tatcccgcga actccgacgc cgacgtcccc 1200
gccgccgttg cccgtctttt tggggatagt cagttcaaca acgggatcga gctgctctcg 1260
gcagccttcg cgaaatggcg aacgccgctt tggagatatc gctttacggg cattccagga 1320
gccggccgtc gccccgccac gcatggagac gaaattccct atgtcttcgc aaatctgggg 1380
ccgtcgtccg tatctatgtt tgggtcgctc gaaggcggcg ccggggcgtc ggacatcaaa 1440
cttgcgaccg aaatgtccgc ggcctgggtg agcttcgcgg tgcacggggt ccccgatcag 1500
ggcacgaaat cgcactggcc gcgcttcgag cggcgagggg agatcatgac ttttggttcg 1560
caggttggct ctggggaagg tcttggagtt tcgccgagca aagcctgcca accctcaaaa 1620
tag 1623
<212> Type : DNA
   <211> Length : 1623
   SequenceName : sequence 8
   SequenceDescription :
Feature
Sequence: sequence 8:
<221> FeatureKey : Seq ID 8 (fumD)
   <222> LocationFrom : 1
   <222> LocationTo : 1623
   Other Information :
   CDSJoin : No
Datebase
Sequence: sequence 8: <308> DBAccessionNumber : FJ426269
   <309> DBEntryDate : 2009-06-11
   <313> From : 1
   <313> To : 1623
Sequence
<213> OrganismName : sphingopyxis sp.
<400> PreSequenceString : 
VKEHQCRGGR ASPAAPATWL ARISVSRGAS AIAWTFMLGA TAIPVAAQTD DPKLVRHTQS 60
GAVEGVEGDV ETFLGIPFAA PPVGDLRWRP PAPPRAWAGT RDGRRFAPDC IGNERLREGS 120
RAAGTSEDCL YLNIWSPKQV GKGGLPVMIW VYGGGFSGGS GAVPYYDGSA LAQKGVVVVT 180
FNYRAGILGF LAHPALSKES PNGVSGNYGL LDMLAAFKWV QNNIREFGGD PNRVTVFGES 240
AGASALGLLL TSPLSESAFN QAILQSPGLA RPLATLSESE ANGLELGADI SALRRADAGE 300
LTKIAQSRIP MSRQFTKPRP MGPILDGYVL RTLDVDAFAK GAFRKIPVLV GGNADEGRAF 360
TDRLPVKTVL EYRAYLTEQF GDEADAWERC YPANSDADVP AAVARLFGDS QFNNGIELLS 420
AAFAKWRTPL WRYRFTGIPG AGRRPATHGD EIPYVFANLG PSSVSMFGSL EGGAGASDIK 480
LATEMSAAWV SFAVHGVPDQ GTKSHWPRFE RRGEIMTFGS QVGSGEGLGV SPSKACQPSK 540
<212> Type : PRT
   <211> Length : 540
   SequenceName : sequence 9
   SequenceDescription :
Feature

sequence: sequence 9: <221> FeatureKey : Seq ID 9 (FumD)
   <222> LocationFrom : 1
   <222> LocationTo : 540
   Other Information :
   CDSJoin : No
Datebase
Sequence: sequence 9:
<308> DBAccessionNumber : FJ426269
   <309> DBEntryDate : 2009-06-11
   <313> From : 1
   <313> To : 540
Sequence
<213> OrganismName : Sphingopyxis sp.
<400> PreSequenceString : 
ttggagtttc gccgagcaaa gcctgccaac cctcaaaata gcgcccggcc tgtgcgtgct 60
tcagcacgcc gtcccgcttt gcgggcgacg ggctgtgccc tctgcctaga aggaagtaag 120
ttgcgctacg acgtcgcgat aattggaggt ggcaacgctg cattgacggc agccgtgacg 180
gcgcgtgaag cgggggcctc ggttcttgtg atcgagcatg cgccgcgcgc catgcgcggc 240
ggcaacagtc gtcacacacg caatatgcgt acgatgcacg aacgtcccct gtcgccgttg 300
accggtgaat attcggcgga cgaatattgg aatgatcttg tccgcgtcac gggggggcgc 360
accgacgaag aactcgcgcg gctcgttatc cgcaacacca ccgacgctat tcccttcatg 420
acgcgctgcg gtgtgcgttt ccagccctcg ctgtcgggca cgctgagttt atcgcgaacc 480
aacgcattct tccttggcgg cgggaaggcg cttgtaaacg catattacgc cacggccgaa 540
cggctaggcg tcgatattct ctatgattct gaggtgaccg agatcaacct tcagcaaggc 600
gtcgtgcagc gtctgcaatt gcgcagccgg ggattccctg tcgaagtgga agccaaggct 660
gccatcgcct cgtccggagg attccaggca aatcttgact ggctctcaag cgcatggggg 720
cctgctgcgg cgaacttcat cgtacggggc acgccatatg cgactggcac ggtgctcaag 780
aacctgttgg agcaaggcgt cgcctcggtg ggagatccaa cccaatgcca tgctgtcgcg 840
atcgatgggc gagcgcccaa atacgacggc ggcatcgtca cacgactgga ctgcgttccc 900
ttctcgatcg tcgtcaacaa ggacgccttg cgcttctacg atgaaggcga agatgtgtgg 960
ccgaagcgtt acgccatatg gggtcgcttg gtggcacagc agcctgatca gatcgctttc 1020
agcataatcg atcggcaggc cgaagacctc ttcatgccgt cagtgttccc ccccgtgcaa 1080
gcggacacga tcgcgggtct ggccgagaaa ctcggtctga atcccgtaac cctggaacgc 1140
acggtggccg aattcaacgc cgcatgcgtg cccggcgaat tcggcggcca agatctcgac 1200
gacctccaca ccgagggaat cgaaccaaag aaatccaact gggcccgacc gattattgtg 1260
cccccgttca gcgcctatcc tctccggccc gggatcacct tcacctatct cggcgtcaag 1320
gtagacagcc gtgcgcgggt catcatggag acaggtgagc cgacaaaaaa cctgtttgct 1380
tcgggggaaa taatggcggg cagcattctc ggccaaggtt atctcgctgg atttggaatg 1440
gcgattggta ccgtattcgg ccgcatcgcg ggttgggagg ccgcacgtca tgcaggattt 1500
tga 1503
<212> Type : DNA
   <211> Length : 1503 SequenceName : sequence 10 SequenceDescription :
Feature
sequence: sequence 10:
<221> FeatureKey : Seq ID 10 (fumE)
   <222> LocationFrom : 1
   <222> LocationTo : 1503 Other Information : CDSJoin : No
Datebase
sequence: sequence 10:
<308> DBAccessionNumber : FJ426269
   <309> DBEntryDate : 2009-06-11
   <313> From : 1
   <313> To : 1503
Sequence
<213> OrganismName : Sphingopyxis sp.
<400> PreSequenceString : 
<400> PreSequenceString :
LEFRRAKPAN PQNSARPVRA SARRPALRAT GCALCLEGSK LRYDVAIIGG GNAALTAAVT 60
AREAGASVLV IEHAPRAMRG GNSRHTRNMR TMHERPLSPL TGEYSADEYW NDLVRVTGGR 120
TDEELARLVI RNTTDAIPFM TRCGVRFQPS LSGTLSLSRT NAFFLGGGKA LVNAYYATAE 180
RLGVDILYDS EVTEINLQQG VVQRLQLRSR GFPVEVEAKA AIASSGGFQA NLDWLSSAWG 240
PAAANFIVRG TPYATGTVLK NLLEQGVASV GDPTQCHAVA IDGRAPKYDG GIVTRLDCVP 300
FSIVVNKDAL RFYDEGEDVW PKRYAIWGRL VAQQPDQIAF SIIDRQAEDL FMPSVFPPVQ 360
ADTIAGLAEK LGLNPVTLER TVAEFNAACV PGEFGGQDLD DLHTEGIEPK KSNWARPIIV 420
PPFSAYPLRP GITFTYLGVK VDSRARVIME TGEPTKNLFA SGEIMAGSIL GQGYLAGFGM 480
AIGTVFGRIA GWEAARHAGF 500
<212> Type : PRT
   <211> Length : 500
   SequenceName : sequence 11
   SequenceDescription :
Feature
Sequence: sequence 11:
<221> FeatureKey : seq ID 11 (FumE)
   <222> LocationFrom : 1
   <222> LocationTo : 500
   Other Information ;
   CDSJoin : No
Datebase
Sequence: sequence 11:
<308> DBAccessionNumber : FJ426269
   <309> DBEntryDate : 2009-06-11
   <313> From : 1
   <313> To : 500
Sequence
<213> OrganismName : Sphingopyxis sp.
<400> PresequenceString : 
atgcaggatt ttgatctcgt aaaaatgctg tctgacttgc cgtcggcgcc ggagctggaa 60
gccaggcgcg ttatggaggt gtgcaacgcg tgccgctatt gcgaagggtt ctgcgcggta 120
tttcctgcaa tgaccttgca gcgtcatttc gccagcggcg atctcagcca cctcgccaat 180
ctctgccact cgtgccaagg ttgctattac gcctgccaat acgcccctcc gcatgagttc 240
ggaataaacg ttccaaaggc gctgtcggag ttgcggctcg agagctacga gcagcatgct 300
tggccccggc cggtcgccgc tctctatcgc aagaatgcgc tcatcatttc catcttgtcg 360
gcggcatgca taaccggcgt ccttctgctt gccgccatct tcaacgggga tgcacttttc 420
gcgaaacacg catcggtgcc cggcggcggg ttttacaacg ttattcctta tcaggcgatg 480
attgccgtcg cggcgaccac atttctttat tccgcgctgg cgctggcgat cagtctcgtt 540
cgcttttcgc ggacgatcgg tctgggaatt aaggttcttt atcagcacgt gccggttctt 600
cgggcgctac gcgatgcggc gactctgcga tatctcggcg gcagcgacgg cgagggggtgt 660
aacgacgcgg acgagacatt ttcgacgacc cggcgaaaat ttcatcacgc ccttgcctat 720
ggcttcggac tttgtttcgc ggccacagcc acgggcacga tctacgatca tatgttcggc 780
tggccggcgc cctatgcgct tttcagcttg ccggtcgtcc tagggaccgt tgggggggatc 840
ggaatggtcg tgggcgcgat cggcctactc tggctcaagc tggccggcga agacgctcct 900
cgatcaccgg cactgcttgg gccggatgtt gccctgttgg tgcttctgct tgccatagcg 960
gcaacgggcc tcctcctttt agcggtccgc agcaccgaag tcatgggcgt cgcgctcgcc 1020
gtccatctcg gcgtcgtctt ggccttcttt ttggtgatgc catacagcaa atttgtccac 1080
ggtatcttca ggctcacggc tctcgtgcgc catcatgctg accgcgaggc aagtaatggc 1140
ttcgcctcca gccctcccac gaaaaagggt taa 1173
<212> Type : DNA
   <211> Length : 1173
   SequenceName : sequence 12
   SequenceDescription :
Feature
Sequence: sequence 12:
<221> FeatureKey : Seq ID 12 (fumF)
   <222> LocationFrom : 1
   <222> LocationTo : 1173
   Other information :
   CDSJoin : No
Datebase
Sequence: sequence 12:
<308> DBAccessionNumber : FJ426269
   <309> DBEntryDate : 2009-06-11
   <313> From : 1
   <313> To : 1173
sequence
<213> OrganismName : Sphingopyxis sp.
<400> PreSequenceString : 
MQDFDLVKML SDLPSAPELE ARRVMEVCNA CRYCEGFCAV FPAMTLQRHF ASGDLSHLAN 60
LCHSCQGCYY ACQYAPPHEF GINVPKALSE LRLESYEQHA WPRPVAALYR KNALIISILS 120
AACITGVLLL AAIFNGDALF AKHASVPGGG FYNVIPYQAM IAVAATTFLY SALALAISLV 180
RFSRTIGLGI KVLYQHVPVL RALRDAATLR YLGGSDGEGC NDADETFSTT RRKFHHALAY 240
GFGLCFAATA TGTIYDHMFG WPAPYALFSL PVVLGTVGGI GMVVGAIGLL WLKLAGEDAP 300
RSPALLGPDV ALLVLLLAIA ATGLLLLAVR STEVMGVALA VHLGVVLAFF LVMPYSKFVH 360
GIFRLTALVR HHADREASNG FASSPPTKKG 390
<212> Type : PRT
   <211> Length : 390
   SequenceName : sequence 13
   SequenceDescription :
Feature
Sequence: sequence 13:
<221> FeatureKey : Seq ID 13 (FumF)
   <222> LocationFrom : 1
   <222> LocationTo : 390
   Other Information :
   CDSJoin : No
Datebase
Sequence: sequence 13:
<308> DBAccessionNumber : FJ426269
   <309> DBEntryDate : 2009-06-11
   <313> From : 1
   <313> To : 390
Sequence
<213> OrganismName : Sphingopyxis sp.
<400> PresequenceString : 
atggaacata tgaagtccgt tcgcgatcgc agtagcgtca tgcagatcgt gagagtggcg 60
agtggcaact gtctcgagca atatgatttc ttcgtttacg gcttctatgc ggcatatatt 120
gcgagaagct tttttccgac cggcgataac gcgacatcgc tcatgctttc attggccact 180
tttggcgctg gtttcctcat gaggcccttg ggggcgattt ttctcgggtc ctacatcgat 240
cgcgtcgggc gtcggaaagg cctgatcgtg acactcgcga tcatggccgt cggaaccctc 300
accattgcga tgactccaag ctatgaggca attggattac tcgcaccggt tatcgtgctc 360
gtcgggcgac ttttgcaggg tttttccgct ggagcagagt cgggtggcgt ctcagtgtac 420
ttggcggaaa ttgcgtcgcc caaatcgaga ggcttcttca cctcgtggca gtctgccagc 480
cagcaggtgg ccgtcatgat cgccgccgcg atcggtcttg cgctgcaatc aacgctttca 540
ccggagcaaa tgaacgactg gggatggcgg gtgcccttgt tgatcggatg cttgattatc 600
cccgtgatac tctggctgcg ccggtctctc ccggaaacga aagcctatct ccacatggag 660
cacaaggcgc attcgatcgg cgaatccctc cgcgaattgc aacagagctg ggggctgatc 720
ttgacgggca tggcgatgtc gatcctcacg acgaccacct tttacatgat taccgcctat 780
acgccgacat ttggcgagaa agcactcgga ctgagcccgc aagatgtcct gctggttacc 840
atcatggtcg gcgtgtcgaa cttcctgtgg cttccgatcg gggtgctct ctcggatcgt 900
atcggtagaa ccccgatcct actggtcgtg ccggtcaccg ttctcgccat cgcctttccc 960
ctgatgagct ggctcgtcgc ggcaccgaca ttcggagcgc ttgcagctgt tctgctgact 1020
ttctccgcat gctttggact ctataatggg gcgctcatcg cgagactcac cgagattatg 1080
cctcccgcca ttagaaccct tggcttctcg ctggcgttca gtctcgcgac ctcgctgttc 1140
ggcggcttca ccccattggt aagtacggcg ctaatccacg cgacgggcag caattccgcg 1200
cctgcaatct ggctctgttt tgcggctttc atcagcttcg tcggtgtggc cgcatcgacc 1260
cggctgagcc ggccaatcgc cgaaggcgcc agatag 1296
<212> Type : DNA
   <211> Length : 1296
   SequenceName : sequence 14
   SequenceDescription :
Feature
Sequence: sequence 14:
<221> FeatureKey : Seq ID 14 (fumG)
   <222> LocationFrom : 1
   <222> LocationTo : 1296 Other Information :
   CDSJoin : No
Datebase
Sequence: sequence 14:
<308> DBAccessionNumber : FJ426269
   <309> DBEntryDate : 2009-06-11
   <313> From : 1
   <313> To : 1296
Sequence
<213> OrganismName : sphingopyxis sp.
<400> PresequenceString : 
MEHMKSVRDR SSVMQIVRVA SGNCLEQYDF FVYGFYAAYI ARSFFPTGDN ATSLMLSLAT 60
FGAGFLMRPL GAIFLGSYID RVGRRKGLIV TLAIMAVGTL TIAMTPSYEA IGLLAPVIVL 120
VGRLLQGFSA GAESGGVSVY LAEIASPKSR GFFTSWQSAS QQVAVMIAAA IGLALQSTLS 180
PEQMNDWGWR VPLLIGCLII PVILWLRRSL PETKAYLHME HKAHSIGESL RELQQSWGLI 240
LTGMAMSILT TTTFYMITAY TPTFGEKALG LSPQDVLLVT IMVGVSNFLW LPIGGALSDR 300
IGRTPILLVV PVTVLAIAFP LMSWLVAAPT FGALAAVLLT FSACFGLYNG ALIARLTEIM 360
PPAIRTLGFS LAFSLATSLF GGFTPLVSTA LIHATGSNSA PAIWLCFAAF ISFVGVAAST 420
RLSRPIAEGA R 431
<212> Type : PRT
   <211> Length : 431
   SequenceName : sequence 15
   SequenceDescription :
Feature
Sequence: sequence 15:
<221> FeatureKey : Seq ID 15 (FumG)
   <222> LocationFrom : 1
   <222> LocationTo : 431
   Other Information :
   CDSJoin : No
   Datebase
Sequence: sequence 15:
<308> DBAccessionNumber : FJ426269
   <309> DBEntryDate : 2009-06-11
   <313> From : 1
   <313> To : 431
Sequence
<213> OrganismName : Sphingopyxis sp.
<400> PreSequenceString : 
<400> PreSequenceString :
atgagagcag tagtttaccg aaatggcgaa cttgtcctgg gggcctatgc tgatccgata 60
cccgccgccg ggcaggtgct cgtcaagacc agagcatgcg gcatctgcgg atctgacctt 120
catttttgcg atcatgcgca ggcgtttacg aaccttgcat cgcgggcggg tatcgcctct 180
atggaagttg atttgtgtcg agacatcgtt ctggggcatg aattctgtgg cgagattatg 240
gagttcgggc cctctgcgga tcgtcgcttc aaacccggac agcttgtgtg ctcgctgccg 300
ctggcgatcg gtccgaccgg agcgcggacg attggctact cggatgagta tcccggcggg 360
ctcggcgaat atatggtcct cacggaagcg ctcttgctgc ctgttccgaa cggccttccg 420
gcgacctgcg cggcgttgac ggagccgatg gcggtgggat ggcatgccgt cgagatcgcg 480
caggttcaac cacatcacat ccctgtggtg atcgggtgcg gaccggtcgg gttggcagtc 540
gtcgctgccc tgaaacataa gcaagttgct ccgattattg cgtcggatcc atcgcccgat 600
cggcgtgctc ttgctctgcg gatgggcgcc gacgccgttg tcgatccgcg cgaagaatca 660
ccctttcgcc aggccgagaa gatcgcacgc ccggtcggac aaggtggggc cctgtccagc 720
tcattgctgt caaagtctca aatgatattc gaatgcgtag ggtgccgggg catgcttcgg 780
catgcgatgg acggcgcgtc cgacgggtcc gagatcatgg tcgttggcgc atgcatgcag 840
ccggacgcga tcgagcccat gatcgggatg tttaaagcgc tcacgatcaa attctcgcga 900
acttacacgg gtgaggaatt cgccgcggtg cttcacatga taggtgaggg cgcactcgac 960
gtatctccgc tcgttaccga tgtgattggc ctgtccgatg tcccgtccgc gtttgaggct 1020
ctacggagtc caggcgccca agcaaaagtg attgtggacc cttggcgctg a 1071
<212> Type : DNA
   <211> Length : 1071
   SequenceName : sequence 16
   SequenceDescription :
Feature
Sequence: sequence 16:
<221> FeatureKey : Seq ID 16 (fumH)
   <222> LocationFrom : 1
   <222> LocationTo : 1071
   Other Information :
   CDSJoin : No
Datebase
Sequence: sequence 16:
<308> DBAccessionNumber : FJ426269
   <309> DBEntryDate : 2009-06-11
   <313> From : 1
   <313> To : 1071
Sequence
<213> OrganismName : Sphingopyxis sp.
<400> PreSequenceString : 
MRAVVYRNGE LVLGAYADPI PAAGQVLVKT RACGICGSDL HFCDHAQAFT NLASRAGIAS 60
MEVDLCRDIV LGHEFCGEIM EFGPSADRRF KPGQLVCSLP LAIGPTGART IGYSDEYPGG 120
LGEYMVLTEA LLLPVPNGLP ATCAALTEPM AVGWHAVEIA QVQPHHIPVV IGCGPVGLAV 180
VAALKHKQVA PIIASDPSPD RRALALRMGA DAVVDPREES PFRQAEKIAR PVGQGGALSS 240
SLLSKSQMIF ECVGVPGMLR HAMDGASDGS EIMVVGACMQ PDAIEPMIGM FKALTIKFSR 300
TYTGEEFAAV LHMIGEGALD VSPLVTDVIG LSDVPSAFEA LRSPGAQAKV IVDPWR 356
<212> Type : PRT
   <211> Length : 356
   SequenceName : sequence 17
   SequenceDescription :
Feature
Sequence: sequence 17:
<221> FeatureKey : Seq ID 17 (FumH)
   <222> LocationFrom : 1
   <222> LocationTo : 356
   Other Information :
   CDSJoin : No
Datebase
Sequence: sequence 17:
<308> DBAccessionNumber : FJ426269
   <309> DBEntryDate : 2009-06-11
   <313> From : 1
   <313> To : 356
Sequence
<213> OrganismName : Sphingopyxis sp.
<400> PreSequenceString : 
atggcgaacg gaacaaggca gaaagatctc agagaacgcg ccgaacgggt cattccgggc 60
gggatgtacg gccacgagtc gacacggttg ctgccgccag aattccccca gttcttcagg 120
cgcgcgctgg gggcacgaat ttgggacgcc gacgagcagc cctatatcga ctatatgtgc 180
gcgtatgggc caaatttgct cggttaccgg caatccgaaa tcgaagccgc ggctgatgcg 240
cagcgacttc tcggcgacac catgaccggt ccttcggaga tcatggtcaa cctcgccgaa 300
gcctttgtgg gcatggtccg tcatgcggat tgggcgatgt tctgcaaaaa tggcagcgat 360
gccacctcaa cggcgatggt tctcgcgcgt gcccatacgg ggcgcaaaac catattatgc 420
gccaaaggcg cctatcatgg cgcttccccg tggaacactc cgcatactgc cgggattctc 480
gcttccgatc gcgtgcatgt cgcatattat acctataacg acgcccaaag cttatcggac 540
Seite 14
 
gcgttcaagg cgcacgatgg cgatattgcg gctgtctttg ccacaccttt ccgacacgaa 600
gtatttgagg accaggccct cgcccagctt gagttcgcgc gcaccgctcg aaaatgttgt 660
gacgagaccg gtgcgcttct ggtcgttgac gatgtgcgcg caggtttccg ggtggcgcgc 720
gattgcagct ggacgcattt gggtatcgaa cccgatctca gttgctgggg aaaatgcttt 780
gcgaatggct atccgatctc cgccctgctg ggctcgaaca aggcgcgcga tgcggcgcgg 840
gatatatttg tgaccggctc cttctggttc tctgcggtac cgatggcggc cgcgatcgaa 900
accctcagga tcattcgaga gacgccttat ctcgaaacgc tgatcgccag cggcgccgcc 960
ctgcgggcag gcctggaggc acagtctcag cgccatggtc ttgagttgaa gcagacgggc 1020
ccggcgcaga tgccgcaaat attctttgcg gacgatcccg attttcggat cggctatgcg 1080
tgggccgcgg cgtgcctgaa gggcggcgtc tatgttcatc cctatcacaa tatgtttctc 1140
tctgcggccc atacagttga cgatgtaacg gagaccctcg aggcgacgga tcgcgcgttc 1200
agcgcggtcc tcagagattt tgcgtctctc cagcctcatc ccattttaat gcaactcgcc 1260
ggtgcttga 1269
<212> Type : DNA
   <211> Length : 1269
   SequenceName : sequence 18
   SequenceDescription :
   Feature
sequence: sequence 18:
<221> FeatureKey : Seq ID 18 (fumI)
   <222> LocationFrom : 1
   <222> LocationTo : 1269
   Other Information :
   CDSJoin : No
Datebase
Sequence: sequence 18:
<308> DBAccessionNumber : FJ426269
   <309> DBEntryDate : 2009-06-11
   <313> From : 1
   <313> To : 1269
Sequence
<213> OrganismName : Sphingopyxis sp.
<400> PreSequenceString : 
MANGTRQKDL RERAERVIPG GMYGHESTRL LPPEFPQFFR RALGARIWDA DEQPYIDYMC 60
AYGPNLLGYR QSEIEAAADA QRLLGDTMTG PSEIMVNLAE AFVGMVRHAD WAMFCKNGSD 120
ATSTAMVLAR AHTGRKTILC AKGAYHGASP WNTPHTAGIL ASDRVHVAYY TYNDAQSLSD 180
AFKAHDGDIA AVFATPFRHE VFEDQALAQL EFARTARKCC DETGALLVVD DVRAGFRVAR 240
DCSWTHLGIE PDLSCWGKCF ANGYPISALL GSNKARDAAR DIFVTGSFWF SAVPMAAAIE 300
TLRIIRETPY LETLIASGAA LRAGLEAQSQ RHGLELKQTG PAQMPQIFFA DDPDFRIGYA 360
WAAACLKGGV YVHPYHNMFL SAAHTVDDVT ETLEATDRAF SAVLRDFASL QPHPILMQLA 420
GA 422
<212> Type : PRT
   <211> Length : 422
   SequenceName : sequence 19
   SequenceDescription :
Feature
Séquence: sequence 19:
<221> FeatureKey : Seq ID 19 (FumI)
   <222> LocationFrom : 1
   <222> LocationTo : 422
   Other Information :
   CDSJoin : No
Datebase
Sequence: sequence 19:
<308> DBAccessionNumber : FJ426269
   <309> DBEntryDate : 2009-06-11
   <313> From : 1
   <313> To : 422
Sequence
<213> OrganismName : Sphingopyxis sp.
<400> PreSequenceString : 
atgtatcgga agttcagaat cgaaaagccc ggcaaggcaa atagtttgct cggcgcagta 60
gcgctcggca ccctcgcatt tcctgtctct gccagtgctc aggatagcga tcccgcatcg 120
ataggtcagc cggacgaagc ggacacggac cggggaacga gcgaaatcgt cgtgaccggc 180
agccgcctcc agaacggctt caattcgccg acgccggtta cagccgtatc cagcgagcag 240
ttgaaggagg catctccgac caaccttgcc gacgcactca accagctgcc cgtgttcaac 300
gacagcttga agacctccaa ccctggcacg acacccggaa cggggaacag cggtcagaac 360
ctgctcaaca tgcgcggcct cgggtcaaac cggaacctcg tcctgctgaa cggcaaccgt 420
ttcgtcgcga ccaatttcac aggctcggtc gatatcaacg tgctgccgca ggcgttggtc 480
aagcgcgtcg atgtcgtgac gggcggcgcc tcggccgcct acggttccga tgccgtttcg 540
ggcgtcatca acttcgtgct cgacgaagat ctggaaggca tcagggccga gctccagtcg 600
ggtgtttcaa cccgcggcga cctcccgtcc tacggcggtt cgatcgcctt cggcacttcg 660
tttgccgacg accggttgca cttgctcggc agcttcgaat attttcgaca ggacggaatc 720
cgggccgatg aagcaacggg tcgccgctgg ttcgacatcg ccgccggcca atatcccgtg 780
cccggcgcta cgacaggcgt cacggtcgtg cccgatattc gcagttctcg cggatcctac 840
ggcggacttg tcacgtccgg ccctctgaaa ggcatcgcgt ttttgcccgg aggagtccta 900
gggaccttcg actacgggaa ttttacgagc tcgtcgttcc agagcggcgg cgatggaccg 960
cgcgtgaata tcggcttcgc cccggatcag cttcgctaca acgcgttcct acgcgccgca 1020
tatgatgtgt ccgacactgt gcaggtgtat gcggagggca cctatgctta ttcccacacc 1080
aacctgggtg cattcgtaat atcgcatgtc ggtggctcga ataatttccg gatcttccgt 1140
gataacgcct tccttccggc tccactcgcg acgctcatgg acagaaatgc ccaggcttcg 1200
atcgttgtcg gtcgcttctc aagcgacttt cccttggtcg aaatcgagaa tttcgcaaag 1260
gtctaccgcg gcgctgccgg cttccgggca gacattggca atggctggaa actcgatggc 1320
tcggcctcct ttggccttac ggacctcgag cttcgtgaaa acaatctcac catcaaccgc 1380
aatctctacg ccgccgtcga tgcggtccgc gatcccgcgg gcaatatcgt ctgccgttca 1440
acactggccg gcctcgacca agattgcgtg ccgctcaatc tcttcggcac aggctcgccg 1500
agcgcgtcgg ccatcgacta tgtcaccgct gatggcgtcg ctcagctgag gcttgagcaa 1560
tatgtggcgg gactcacgat ttccggcgac ctcggcgata gcctgtcgtt cggcgcgggc 1620
ccggtctcgg tcgccgctgg tatcgaatat cgcaaggaga aggcccggca ggaaaccgac 1680
gcgatatcgc aggcgacgac ctcgatcacg ggaatcaggg gggctccggc ggcgcaggca 1740
ggtcggcctg gaggcttcaa tctctacaac ccacttccct tctcggaagg ctatgacatc 1800
aaggaaggtt ttgtcgaaat cggcgtcccg attctgaagg acagcgcgct gggacgttcg 1860
ctgaacttaa acggcgccgt ccgatatgcc gattacagcc agtccggtgg agtaacaacc 1920
tggaagctgg gcggagaata tgagccgatc gacggcctca ggttccgcgc gacccgttcg 1980
cgagatatcc gcgggccaag ccttgtcgag ctattcgacc ccggccgtca ggcgacgctc 2040
aattcaattt atggcggaca ggctgtgcag acgcggttct ttaccgccgg caacgcggat 2100
ttgcgccctg aaaaggcgga cgtccttaca ttcggcgcgg tgctacgccc cgccttcgtg 2160
ccggggtttc agttttcggt cgatcgctat gtggtgaagg tgaagggcgc gatcgatttc 2220
ctccttcccc agcaggaaat cgacgcgtgc gatgcaggaa acaccttctt ctgcgacctc 2280
ataacggaga atccggacgg caccatcaca gtgacgggtc ccaatctcaa cctggctgtc 2340
cagaaagcgg cgggaattga cttcgaggcc tattactcac gccccgtcgg cggcggcacg 2400
ttcagtcttc gtgcgctggc aacgcaccat acctctgcct atcgcatcgc gaccggctcg 2460
gcgcccatcc gttcgctcgg acaaccggac acgccaaaat ggtcggccaa cttccaggcg 2520
cgatattcga ccgacgattg ggcgcttctc gtgcagcagc gcttcatcgc agcatcggtg 2580
ttcaatgccg acaatgtgga gggcgtcgat acgaatttga accacgctcc ggcggtttgg 2640
tacaccgacg cgacattgac cttcgacatc gcggcttttg gccagaagca gcagctgttt 2700
ctatcggtca ataatttgtt cgaccgagat ccgccaatag cgacgaacga ccccagcagt 2760
ttttccagcc cgaccagctc tgcctatgat ccggtcggcc gctattttaa tgtcggggtc 2820
cgtttccgga tctga 2835
<212> Type : DNA
   <211> Length : 2835
   SequenceName : sequence 20
   SequenceDescription :
Feature
sequence: sequence 20:
<221> FeatureKey : Seq ID 20 (fumJ)
   <222> LocationFrom : 1
   <222> LocationTo : 2835
   Other Information :
   CDSJoin : No
Datebase
Sequence: sequence 20:
<308> DBAccessionNumber : FJ426269
   <309> DBEntryDate : 2009-06-11
   <313> From : 1
   <313> To : 2835
Sequence
<213> OrganismName : Sphingopyxis sp.
<400> PreSequenceString : 
MYRKFRIEKP GKANSLLGAV ALGTLAFPVS ASAQDSDPAS IGQPDEADTD RGTSEIVVTG 60
SRLQNGFNSP TPVTAVSSEQ LKEASPTNLA DALNQLPVFN DSLKTSNPGT TPGTGNSGQN 120
LLNMRGLGSN RNLVLLNGNR FVATNFTGSV DINVLPQALV KRVDVVTGGA SAAYGSDAVS 180
GVINFVLDED LEGIRAELQS GVSTRGDLPS YGGSIAFGTS FADDRLHLLG SFEYFRQDGI 240
RADEATGRRW FDIAAGQYPV PGATTGVTVV PDIRSSRGSY GGLVTSGPLK GIAFLPGGVL 300
GTFDYGNFTS SSFQSGGDGP RVNIGFAPDQ LRYNAFLRAA YDVSDTVQVY AEGTYAYSHT 360
NLGAFVISHV GGSNNFRIFR DNAFLPAPLA TLMDRNAQAS IVVGRFSSDF PLVEIENFAK 420
VYRGAAGFRA DIGNGWKLDG SASFGLTDLE LRENNLTINR NLYAAVDAVR DPAGNIVCRS 480
TLAGLDQDCV PLNLFGTGSP SASAIDYVTA DGVAQLRLEQ YVAGLTISGD LGDSLSFGAG 540
PVSVAAGIEY RKEKARQETD AISQATTSIT GIRGAPAAQA GRPGGFNLYN PLPFSGSYDI 600
KEGFVEIGVP ILKDSALGRS LNLNGAVRYA DYSQSGGVTT WKLGGEYEPI DGLRFRATRS 660
RDIRGPSLVE LFDPGRQATL NSIYGGQAVQ TRFFTAGNAD LRPEKADVLT FGAVLRPAFV 720
PGFQFSVDRY VVKVKGAIDF LLPQQEIDAC DAGNTFFCDL ITENPDGTIT VTGPNLNLAV 780
QKAAGIDFEA YYSRPVGGGT FSLRALATHH TSAYRIATGS APIRSLGQPD TPKWSANFQA 840
RYSTDDWALL VQQRFIAASV FNADNVEGVD TNLNHAPAVW YTDATLTFDI AAFGQKQQLF 900
LSVNNLFDRD PPIATNDPSS FSSPTSSAYD PVGRYFNVGV RFRI 944
<212> Type : PRT
   <211> Length : 944
   SequenceName : sequence 21
   SequenceDescription :
Feature
sequence: sequence 21:
<221> FeatureKey : Seq ID 21 (FumJ)
   <222> LocationFrom : 1
   <222> LocationTo : 944
   Other Information :
   CDSJoin : No
Datebase
Sequence: sequence 21:
<308> DBAccessionNumber : FJ426269
   <309> DBEntryDate : 2009-06-11
   <313> From : 1
   <313> To : 944
Sequence
<213> OrganismName : Sphingopyxis sp.
<400> PreSequenceString : 
atgcgcctca cggcggaga attattggca cgatgtttgg ccgtcgaagg cgtccggtat 60
gtcttcggcc tcatgtcgcc ggaggtggat ccgctcctgg ctgcgctcga agacaatggg 120
atattgttcg tcccggtgcg gcacgaggcc gccgcagcct atatggccga gggcatttac 180
aagaccaccg gacaggtcgc cgcgattgtc acgaatccgg gtcccggtac ggcaaacctt 240
ctgcctggag tcgtgacggc acgccacgaa ggggttccct tcgtcgcaat aacgtcccag 300
catcaacttg gtgtcgttta tccctgcacg ccaaaaacct ttcagggaca agaccagatc 360
gacctctttc gacccgcggt taaatggggc gcacccatct tcgcctggaa ccggatt 417
<212> Type : DNA
   <211> Length : 417
   SequenceName : sequence 22
   SequenceDescription :
Feature
Sequence: sequence 22:
<221> FeatureKey : seq ID 22 (fumK)
   <222> LocationFrom : 1
   <222> LocationTo : 417
   Other Information :
   CDSJoin : No
Datebase
Sequence: sequence 22:
<308> DBAccessionNumber : FJ426269
   <309> DBEntryDate : 2009-06-11
   <313> From : 1
   <313> To : 417
Sequence
<213> OrganismName : Sphingopyxis sp.
<400> PreSequenceString : 
MRLTGGELLA RCLAVEGVRY VFGLMSPEVD PLLAALEDNG ILFVPVRHEA AAAYMAEGIY 60
KTTGQVAAIV TNPGPGTANL LPGVVTARHE GVPFVAITSQ HQLGVVYPCT PKTFQGQDQI 120
DLFRPAVKWG APIFAWNRI 139
<212> Type : PRT
   <211> Length : 139
   SequenceName : sequence 23
   SequenceDescription :
Feature
Sequence: sequence 23:
<221> FeatureKey : Seq ID 23 (FumK)
   <222> LocationFrom : 1
   <222> LocationTo : 139
   Other Information :
   CDSJoin : No
Datebase
Sequence: sequence 23:
<308> DBAccessionNumber : FJ426269
   <309> DBEntryDate : 2009-06-11
   <313> From : 1
   <313> To : 139
Sequence
<213> OrganismName : Caulobacter sp.
<400> PreSequenceString : 
atggaattga gccgccaacg agaccaggcc ttgagggagc gcgcccaagc ggtgatcccg 60
ggcgggatgt acggtcacga gtcgacctat ctgatgcccg agggcacgcc acagttcttc 120
agtcgcggca aaggcgcccg actttgggac gccgacggca acgagtatgt cgattacatg 180
tgcgcctatg gccccaacct gctgggttac ggcttcgaac ccgtcgaagc ggccgccgca 240
gcccagcaag cccggggcga taccctgacc gggccgtcgg aggtgatggt gcagttggcg 300
gaagacttcg tcgcgcaaat cagccacgcg gactgggcca tgttctgcaa gaacggcaca 360
gacgccacct caatggcgat ggtcatcgcg cgcgcacaca ccggccggaa gacgatcctc 420
tgcgcgaaag gcgcctatca tggggccgcg ccttggtgca cgccgatcct ggccggaacg 480
ctaccggagg atcgcgcctt tgtagtctac tacgactaca atgacgccca aagcctcgtc 540
gacgccttcg aggcccatca ggacgacgtc gcggcgatct tcgccacccc tcaccgtcac 600
gaggtgttca gcgaccagat cgatcctgat ccggaatatg cggccagcgt gcgggcgctc 660
tgcgacaaga gcggcgccct gctcgtcgtc gacgaagttc gagccgggtt caggatcgcg 720
cgcgactgca gctgggccaa gatcggcgtc gctccggatc tgagcacctg gggcaagtgc 780
ttcgccaacg gctatccgat ctcggcggtc ctagggggcg aaaaggtgcg cagcgcggca 840
aaggccgtct acgtcaccgg ctcgttctgg ttctcggcca cgcccatggc cgcagccgtc 900
gaaaccctga agcaaatccg cgagaccgac tatctcgagc ggatcaacgc ggccgggacc 960
cgcctgcgcg agggcctgca gcagcaggct gctcacaacg gctttacgtt gcgccaaacg 1020
gggcccgtct ccatgcccca agtcctcttc gaggaagatc ccgattttcg ggtcggctac 1080
ggctgggttc gcgaatgcct gaagcgaggg gtgtacttca gcccctacca taacatgttc 1140
ctgtcggcgg cccatagcga ggcggacctg gccaagaccc ttgcggctac cggcgacgcc 1200
 
ttcgtcgagc tacgcgccaa gcttccgagc ctagaaatcc accaacccct cctcgccctg 1260
agagcggcct aa 1272
<212> Type : DNA
   <211> Length : 1272
   SequenceName : sequence 24
   SequenceDescription :
Feature
Sequence: sequence 24:
<221> FeatureKey : Seq ID 24
   <222> LocationFrom : 1
   <222> LocationTo : 1272
   Other Information :
   CDSJoin : No
Datebase
sequence: sequence 24:
<308> DBAccessionNumber : FJ426269
   <309> DBEntryDate : 2009-06-11
   <313> From : 1
   <313> To : 1272
Sequence
<213> OrganismName : Caulobacter-sp.
<400> PreSequenceString : 
MELSRQRDQA LRERAQAVIP GGMYGHESTY LMPEGTPQFF SRGKGARLWD ADGNEYVDYM 60
CAYGPNLLGY GFEPVEAAAA AQQARGDTLT GPSEVMVQLA EDFVAQISHA DWAMFCKNGT 120
DATSMAMVIA RAHTGRKTIL CAKGAYHGAA PWCTPILAGT LPEDRAFVVY YDYNDAQSLV 180
DAFEAHQDDV AAIFATPHRH EVFSDQIDPD PEYAASVRAL CDKSGALLVV DEVRAGFRIA 240
RDCSWAKIGV APDLSTWGKC FANGYPISAV LGGEKVRSAA KAVYVTGSFW FSATPMAAAV 300
ETLKQIRETD YLERINAAGT RLREGLQQQA AHNGFTLRQT GPVSMPQVLF EEDPDFRVGY 360
GWVRECLKRG VYFSPYHNMF LSAAHSEADL AKTLAATGDA FVELRAKLPS LEIHQPLLAL 420
RAA 423
<212> Type : PRT
   <211> Length : 423
   SequenceName : sequence 25
   SequenceDescription :
Feature
Sequence: sequence 25:
<221> FeatureKey : Seq ID 25
   <222> LocationFrom : 1
   <222> LocationTo : 423
   Other Information :
   CDSJoin : No
Datebase
Sequence: sequence 25:
<308> DBAccessionNumber : FJ426269
   <309> DBEntryDate : 2009-06-11
   <313> From : 1
   <313> To : 423

## Patentansprüche

1. Zusatzstoff geeignet zum enzymatischen Abbau von Fumonisinen, in einem pflanzlichen Rohstoff und Mischungen, die pflanzliche Rohstoffe enthalten, **dadurch gekennzeichnet, dass** ein Enzym der Sequenz ID-Nr. 9 sowie gegebenenfalls zusätzlich ein Cosubstrat für das eingesetzte Enzym, ein Enzym der Sequenz ID-Nr. 19 und ein inerter Träger enthalten sind.

2. Zusatzstoff nach Anspruch 1, **dadurch gekennzeichnet, dass** das Enzym mit einer Schutzhülle ummantelt eingesetzt ist.

3. Zusatzstoff nach Anspruch 1 oder 2, **dadurch gekennzeichnet, dass** eine Carboxylesterase Sequenz ID-Nr. 9, eine Aminotransferase Sequenz ID-Nr. 19, eine α-Ketosäure als Cosubstrat und ein inerter Träger enthalten sind.

4. Zusatzstoff nach Anspruch 1, 2 oder 3, **dadurch gekennzeichnet, dass** eine Carboxylesterase Sequenz ID-Nr. 9, wenigstens ein Adsorbens, wie ein Tonmineral, sowie ein inerter Träger enthalten sind.

5. Verwendung eines Zusatzstoffes nach einem der Ansprüche 1 bis 4, **dadurch gekennzeichnet, dass** der Zusatzstoff in einem sauerstoffunabhängigen Milieu bei der Bioethanolherstellung, gemeinsam mit einer Maische bzw. einem pflanzlichen Ausgangsmaterial, eingesetzt ist.

6. Verwendung eines Zusatzstoffes nach einem der Ansprüche 1 bis 4, **dadurch gekennzeichnet, dass** der Zusatzstoff in einem zu vergärenden, pflanzlichen Rohstoff bzw. in einer Maische zur Bioethanolherstellung eingesetzt wird.

## Claims

1. An additive for the enzymatic degradation of fumonisins in vegetable raw materials and mixtures containing vegetable raw materials, **characterized in that** it contains an enzyme of the sequence ID No. 9 as well as optionally, in addition, at least a cosubstrate for the used enzyme, and an inert carrier.

2. An additive according to claim 1, **characterized in that** the enzyme covered in a protective shell is used.

3. An additive according to claim 1 or 2, **characterized in that** a carboxylesterase with sequence ID No. 9, an aminotransferase with sequence ID No. 19, an α-keto acid and an inertial carrier are contained.

4. An additive according to claim 1, 2 or 3, **characterized in that** a carboxylesterase with sequence ID No. 9, at least one adsorbent, like a clay material, as well as an inert carrier are contained.

5. Use of an additive according to any one of claims 1 to 4, **characterized in that** the additive is used in an oxygen-independent environment during the production of bioethanol along with a mash or a vegetable starting material.

6. Use of an additive according to any one of claims 1 to 4, **characterized in that** the additive is used in a herbal raw material which is to be fermented or a mash in the production of bioethanol.

## Revendications

1. Additif convenant à la dégradation enzymatique de fumonisines, dans une matière première végétale et dans des mélanges contenant des matières premières végétales, **caractérisé en ce qu'**une enzyme de la séquence ID n° 9 ainsi que le cas échéant en supplément un cosubstrat pour l'enzyme utilisée, une enzyme de la séquence ID n° 19 et un support inerte sont contenus.

2. Additif selon la revendication 1, **caractérisé en ce que** l'enzyme est utilisée sous forme enrobée d'une enveloppe de protection.

3. Additif selon la revendication 1 ou 2, **caractérisé en ce qu'**une carboxylestérase de la séquence ID n° 9, une aminotransférase de la séquence ID n° 19, un α-acide cétonique en tant que cosubstrat et un support inerte sont contenus.

4. Additif selon la revendication 1, 2 ou 3, **caractérisé en ce qu'**une carboxylestérase de la séquence ID n° 9, au moins un adsorbant, tel qu'un minéral d'argile, ainsi qu'un support inerte sont contenus.

5. Utilisation d'un additif selon l'une quelconque des revendications 1 à 4, **caractérisé en ce que** l'additif est utilisé dans un milieu anaérobie lors de la production de bioéthanol, conjointement avec un moût ou un matériau de départ végétal.

6. Utilisation d'un additif selon l'une quelconque des revendications 1 à 4, **caractérisée en ce que** l'additif est utilisé dans une matière première végétale à fermenter ou dans un moût servant à produire du bioéthanol.
